# EUROPEAN PATENT APPLICATION

(11) **EP 4 799 650 A1**
(43) Date of publication of application: **02.09.2026**
(21) Application number: 24881746.2
(22) Date of filing: 25.10.2024
(51) Int. Cl.: A61K 47/68, A61K 31/4745, A61P 35/00

(54) **USE OF ANTI-HER2 ANTIBODY-DRUG CONJUGATE IN TREATMENT OF BREAST CANCER**

(30) Priority: 25.10.2023 CN 202311398629
(71) Applicant: Chia Tai Tianqing Pharmaceutical Group Co., Ltd., Lianyungang, Jiangsu 222062 (CN)
(72) Inventor: FENG, Fan, Lianyungang, Jiangsu 222062 (CN); YU, Ding, Lianyungang, Jiangsu 222062 (CN); WANG, Xunqiang, Lianyungang, Jiangsu 222062 (CN); ZHANG, Xiquan, Lianyungang, Jiangsu 222062 (CN); CHEN, Jie, Lianyungang, Jiangsu 222062 (CN)
(74) Representative: v. Bezold & Partner Patentanwälte - PartG mbB
(86) International application number: PCT/CN2024/127375
(87) International publication number: WO 2025/087386

(57) **Abstract**

The present invention belongs to the field of biomedicine, and relates to use of an anti-HER2 antibody-drug conjugate in the treatment of breast cancer. The present invention also relates to use of the anti-HER2 antibody-drug conjugate in the preparation of a medicament for treating breast cancer in a subject. The present invention further relates to a method for treating breast cancer in a subject, the method comprising administering to the subject the anti-HER2 antibody-drug conjugate. The method provides benefits to breast cancer subjects and demonstrates good safety.

## Description

### TECHNICAL FIELD

The present disclosure pertains to the field of biomedicine, and particularly relates to use of an anti-HER2 antibody-drug conjugate in the treatment of breast cancer.

### BACKGROUND

Human epidermal growth factor receptor 2 (HER2) belongs to the human epidermal growth factor receptor family, which includes EGFR (ErbB-1), HER2/c-neu (ErbB-2), HER3 (ErbB-3), and HER4 (ErbB-4). These receptors are all located on the cell surface and have similar structures. As a widely expressed receptor protein, HER2 exhibits abnormal gene amplification that leads to protein overexpression, which in turn causes abnormal activation of signaling pathways, serving as one of the main pathways for promoting the growth of solid tumors. HER2 can form homodimers and is also prone to forming heterodimers with other receptor members of the HER family, leading to phosphorylation of receptor tyrosine residues and initiation of various signaling pathways, including MPK, PI3K, JAK, STAT3, PKC, and the like, thereby resulting in cell proliferation and tumorigenesis.

Anti-HER2 targeted therapy is an important approach for treating breast cancer; therefore, there is an urgent need to explore drugs targeting HER2 to meet the huge clinical demand for breast cancer treatment.

### SUMMARY

### Methods for Treating Breast Cancer

The present disclosure provides a method for treating breast cancer in a subject, which comprises administering to the subject the anti-HER2 antibody-drug conjugate of the present disclosure. The present disclosure further provides a method for treating breast cancer in a subject in second-line treatment or a later line of treatment, which comprises administering to the subject the anti-HER2 antibody-drug conjugate of the present disclosure. In some embodiments, in the method, the anti-HER2 antibody-drug conjugate is administered in a therapeutically effective amount.

The present disclosure further provides use of the anti-HER2 antibody-drug conjugate of the present disclosure for preparing a medicament for treating breast cancer in a subject. The present disclosure further provides use of the anti-HER2 antibody-drug conjugate of the present disclosure for preparing a medicament for treating breast cancer in a subject in second-line treatment or a later line of treatment. In some embodiments, in the use, the medicament comprises a therapeutically effective amount of the anti-HER2 antibody-drug conjugate.

The present disclosure further provides the anti-HER2 antibody-drug conjugate of the present disclosure for use in treating breast cancer in a subject. The present disclosure further provides the anti-HER2 antibody-drug conjugate of the present disclosure for use in treating breast cancer in a subject in second-line treatment or a later line of treatment. In some embodiments, the anti-HER2 antibody-drug conjugate is administered to the subject in a therapeutically effective amount.

The present disclosure further provides use of the anti-HER2 antibody-drug conjugate of the present disclosure for treating breast cancer in a subject.

In some embodiments, in the method or the use, the anti-HER2 antibody-drug conjugate is administered once every 1 week (q1w), every 2 weeks (q2w), every 3 weeks (q3w), or every 4 weeks (q4w). In one specific embodiment, in the method or the use, the anti-HER2 antibody-drug conjugate is administered once every 3 weeks. In some embodiments, in the method or the use, the anti-HER2 antibody-drug conjugate is administered at a dose of 1.5-9 mg/kg, 4.5-7.5 mg/kg, or 6-7.5 mg/kg each time. In some embodiments, in the method or the use, the anti-HER2 antibody-drug conjugate is administered at a dose of 1.5 mg/kg, 3 mg/kg, 4.5 mg/kg, 6 mg/kg, 7.5 mg/kg, 9 mg/kg, or a range formed by any of the above values each time. In some embodiments, in the method or the use, the anti-HER2 antibody-drug conjugate is administered at a dose of 6 mg/kg or 7.5 mg/kg each time. In some embodiments, in the method or the use, the anti-HER2 antibody-drug conjugate is administered at a dose of 7.5 mg/kg each time. In some embodiments, in the method or the use, the anti-HER2 antibody-drug conjugate is administered at a dose of 6 mg/kg each time. In some embodiments, in the method or the use, the anti-HER2 antibody-drug conjugate is administered once every 1 week, every 2 weeks, every 3 weeks, or every 4 weeks at a dose of 1.5-9 mg/kg, 4.5-7.5 mg/kg, or 6-7.5 mg/kg of the anti-HER2 antibody-drug conjugate each time. In some embodiments, in the method or the use, the anti-HER2 antibody-drug conjugate is administered once every 3 weeks at a dose of 1.5 mg/kg, 3 mg/kg, 4.5 mg/kg, 6 mg/kg, 7.5 mg/kg, or 9 mg/kg of the anti-HER2 antibody-drug conjugate each time. In some embodiments, in the method or the use, the anti-HER2 antibody-drug conjugate is administered once every 3 weeks at a dose of 6 mg/kg or 7.5 mg/kg of the anti-HER2 antibody-drug conjugate each time. In some embodiments, in the method or the use, the anti-HER2 antibody-drug conjugate is administered once every 3 weeks at a dose of 6 mg/kg of the anti-HER2 antibody-drug conjugate each time. In some embodiments, in the method or the use, the anti-HER2 antibody-drug conjugate is administered once every 3 weeks at a dose of 7.5 mg/kg of the anti-HER2 antibody-drug conjugate each time.

In some embodiments, in the method or the use, one treatment cycle comprises 1 week, 2 weeks, 3 weeks, or 4 weeks. In some embodiments, in the method or the use, one treatment cycle comprises 3 weeks. In some embodiments, in the method or the use, one treatment cycle comprises 1 week, 2 weeks, 3 weeks, or 4 weeks, and the anti-HER2 antibody-drug conjugate is administered once per treatment cycle. In some embodiments, in the method or the use, one treatment cycle comprises 3 weeks, and the anti-HER2 antibody-drug conjugate is administered once per treatment cycle. In some embodiments, in the method or the use, one treatment cycle comprises 3 weeks, and 6 mg/kg or 7.5 mg/kg of the anti-HER2 antibody-drug conjugate is administered per treatment cycle. In some embodiments, in the method or the use, one treatment cycle comprises 3 weeks, and 6 mg/kg or 7.5 mg/kg of the anti-HER2 antibody-drug conjugate is administered on day 1 per treatment cycle. In some embodiments, in the method or the use, one treatment cycle comprises 3 weeks, and 6 mg/kg of the anti-HER2 antibody-drug conjugate is administered per treatment cycle. In some embodiments, in the method or the use, one treatment cycle comprises 3 weeks, and 6 mg/kg of the anti-HER2 antibody-drug conjugate is administered on day 1 per treatment cycle. In some embodiments, in the method or the use, one treatment cycle comprises 3 weeks, and 7.5 mg/kg of the anti-HER2 antibody-drug conjugate is administered per treatment cycle. In some embodiments, in the method or the use, one treatment cycle comprises 3 weeks, and 7.5 mg/kg of the anti-HER2 antibody-drug conjugate is administered on day 1 per treatment cycle. In some embodiments, different doses of the anti-HER2 antibody-drug conjugate are administered to the same subject in different treatment cycles. In some embodiments, in the first treatment cycle, the anti-HER2 antibody-drug conjugate is administered to the subject at a dose of 7.5 mg/kg, and in the second and subsequent treatment cycles, the anti-HER2 antibody-drug conjugate is administered to the subject at a dose of 6 mg/kg. In some embodiments, in the first treatment cycle, the anti-HER2 antibody-drug conjugate is administered to the subject at a dose of 6 mg/kg, and in the second and subsequent treatment cycles, the anti-HER2 antibody-drug conjugate is administered to the subject at a dose of 7.5 mg/kg.

The anti-HER2 antibody-drug conjugate may be formulated together with one or more pharmaceutically acceptable excipients to prepare a suitable pharmaceutical composition (or referred to as a formulation). The pharmaceutical composition may be in a suitable dosage form. In some embodiments, the anti-HER2 antibody-drug conjugate is formulated into a formulation for parenteral administration. In some specific embodiments, the anti-HER2 antibody-drug conjugate is formulated into a formulation for intravenous, intramuscular, or other parenteral administration. In some specific embodiments, the anti-HER2 antibody-drug conjugate may be formulated into an injection. In some specific embodiments, the anti-HER2 antibody-drug conjugate is formulated into a formulation for intravenous injection or infusion.

In some embodiments, in the method or the use, the anti-HER2 antibody-drug conjugate is administered by intravenous infusion.

In the method or the use, the administration regimen (e.g., administration cycle, administration time, administration dose, and dose adjustment) of the anti-HER2 antibody-drug conjugate may be adjusted according to the severity of the disease, the response to the disease, any treatment-related toxicity, and the age and health status of a patient. For example, the anti-HER2 antibody-drug conjugate may be administered with a delay of 1-84 days, such as a delay of 1 week, 2 weeks, 3 weeks, 4 weeks, 5 weeks, 6 weeks, 7 weeks, 8 weeks, 9 weeks, 10 weeks, 11 weeks, or 12 weeks. For another example, the administration dose of the anti-HER2 antibody-drug conjugate may be adjusted from 6 mg/kg to 4.5 mg/kg, or from 7.5 mg/kg to 6 mg/kg.

In some embodiments, the breast cancer is unresectable, refractory, advanced, recurrent, and/or metastatic breast cancer. In some embodiments, the breast cancer is unresectable breast cancer. In some embodiments, the breast cancer is refractory breast cancer. In some embodiments, the breast cancer is advanced breast cancer. In some embodiments, the breast cancer is recurrent and/or metastatic breast cancer. In some embodiments, the breast cancer is locally advanced breast cancer. In some embodiments, the breast cancer is locally recurrent breast cancer. In some embodiments, the breast cancer is locally recurrent and/or metastatic breast cancer. In some embodiments, the breast cancer is unresectable recurrent breast cancer. In some embodiments, the breast cancer is unresectable metastatic breast cancer. In some embodiments, the breast cancer is locally advanced or metastatic breast cancer.

In some embodiments, the breast cancer is HER2-expressing breast cancer. In some embodiments, the breast cancer is HER2-positive breast cancer. In some embodiments, the HER2-positive breast cancer is breast cancer determined to have HER2 expression of 3+ by immunohistochemistry (IHC). In some embodiments, the HER2-positive breast cancer is breast cancer determined to have HER2 expression of 2+ by IHC and determined to be positive for HER2 expression by ISH. In some embodiments, the breast cancer is HER2-low expressing breast cancer. In some embodiments, the HER2-low expressing breast cancer is breast cancer determined to have HER2 expression of 2+ by IHC and determined to be negative for HER2 expression by ISH. In some embodiments, the HER2-low expressing breast cancer is breast cancer determined to have HER2 expression of 1+ by IHC.

In some embodiments, the breast cancer is HER2-expressing recurrent and/or metastatic breast cancer. In some embodiments, the breast cancer is HER2-positive recurrent and/or metastatic breast cancer. In some embodiments, the breast cancer is recurrent and/or metastatic breast cancer determined to have HER2 expression of 3+ by IHC, or determined to be positive for HER2 expression by ISH, or determined to have HER2 expression of 2+ by IHC and determined to be positive for HER2 expression by ISH. In some embodiments, the breast cancer is HER2-low expressing recurrent and/or metastatic breast cancer. In some embodiments, the breast cancer is recurrent and/or metastatic breast cancer determined to have HER2 expression of 1+ by IHC, or determined to have HER2 expression of 2+ by IHC and determined to be negative for HER2 expression by ISH.

In some embodiments, the subject with breast cancer is positive for the hormone receptor (HR). In some embodiments, the subject with breast cancer is positive for the estrogen receptor (ER). In some embodiments, the subject with breast cancer is positive for the progesterone receptor (PR). In some embodiments, the breast cancer is hormone receptor (HR)-negative or positive and HER2-positive recurrent or metastatic breast cancer.

In some embodiments, the subject with breast cancer is unsuitable for receiving surgery and/or radiation therapy. In some embodiments, the subject with HER2-expressing breast cancer is unsuitable for receiving surgery and/or radiation therapy. In some embodiments, the subject with recurrent and/or metastatic breast cancer is unsuitable for receiving surgery and/or radiation therapy. In some embodiments, the subject with HER2-expressing recurrent and/or metastatic breast cancer is unsuitable for receiving surgery and/or radiation therapy. In some embodiments, the subject with HER2-positive recurrent and/or metastatic breast cancer is unsuitable for receiving surgery and/or radiation therapy. In some embodiments, the subject with HER2-low expressing recurrent and/or metastatic breast cancer is unsuitable for receiving surgery and/or radiation therapy. Being unsuitable for receiving surgery and/or radiation therapy includes being unable to benefit from surgery and/or radiation therapy, or being unable to be cured by surgery and/or radiation therapy.

In some embodiments, the subject with breast cancer has previously received at least one line of treatment for treating breast cancer (e.g., treatment failure or intolerance). In some embodiments, the subject with breast cancer has previously received at least two lines of treatment for treating breast cancer (e.g., treatment failure or intolerance). In some embodiments, the subject with breast cancer has previously received at least three lines of treatment for treating breast cancer (e.g., treatment failure or intolerance). In some embodiments, the subject with recurrent and/or metastatic breast cancer has previously received at least one line of treatment for treating recurrent and/or metastatic breast cancer (e.g., treatment failure or intolerance). In some embodiments, the subject with recurrent and/or metastatic breast cancer has previously received at least two lines of treatment for treating recurrent and/or metastatic breast cancer (e.g., treatment failure or intolerance). In some embodiments, the subject with recurrent and/or metastatic breast cancer has previously received at least three lines of treatment for treating recurrent and/or metastatic breast cancer (e.g., treatment failure or intolerance). In some embodiments, the subject with HER2-expressing breast cancer has previously received at least one line of treatment for treating HER2-expressing breast cancer (e.g., treatment failure or intolerance). In some embodiments, the subject with HER2-expressing breast cancer has previously received at least two lines of treatment for treating HER2-expressing breast cancer (e.g., treatment failure or intolerance). In some embodiments, the subject with HER2-expressing breast cancer has previously received at least three lines of treatment for treating HER2-expressing breast cancer (e.g., treatment failure or intolerance). In some embodiments, the subject with breast cancer has previously received first-line chemotherapy for treating breast cancer (e.g., treatment failure or intolerance), and the breast cancer is preferably recurrent and/or metastatic breast cancer, and more preferably HER2-expressing recurrent and/or metastatic breast cancer.

In some embodiments, the subject with breast cancer has previously received at least one line of systemic chemotherapy for treating breast cancer (e.g., treatment failure or intolerance). In some embodiments, the subject with recurrent and/or metastatic breast cancer has previously received at least one line of systemic treatment for treating breast cancer (e.g., treatment failure or intolerance). In some embodiments, the subject with HER2-expressing recurrent and/or metastatic breast cancer has previously received at least one line of systemic treatment for treating breast cancer (e.g., treatment failure or intolerance). In some embodiments, the subject with HER2-low expressing recurrent and/or metastatic breast cancer has previously received at least one line of systemic treatment for treating breast cancer (e.g., treatment failure or intolerance).

In some embodiments, the subject with breast cancer has previously received adjuvant therapy for treating breast cancer (e.g., treatment failure or intolerance). In some embodiments, the subject with breast cancer has previously received adjuvant therapy for treating breast cancer and experienced treatment failure. In some embodiments, the subject with breast cancer has experienced disease recurrence during the period of receiving adjuvant therapy for treating breast cancer. In some embodiments, the subject with breast cancer has experienced disease recurrence after receiving adjuvant therapy for treating breast cancer. In some embodiments, the subject with breast cancer has previously received adjuvant therapy for treating breast cancer and experienced disease recurrence within 6 months (e.g., 1, 2, 3, 4, 5, or 6 months) after the end of the treatment. In some embodiments, the subject with recurrent and/or metastatic breast cancer has previously received adjuvant therapy for treating breast cancer and experienced treatment failure. In some embodiments, the subject with recurrent and/or metastatic breast cancer has experienced disease recurrence during the period of receiving adjuvant therapy for treating breast cancer. In some embodiments, the subject with recurrent and/or metastatic breast cancer has experienced disease recurrence after receiving adjuvant therapy for treating breast cancer. In some embodiments, the subject with recurrent and/or metastatic breast cancer has previously received adjuvant therapy for treating breast cancer and experienced disease recurrence within 6 months (e.g., 1, 2, 3, 4, 5, or 6 months) after the end of the treatment. In some embodiments, the subject with HER2-expressing recurrent and/or metastatic breast cancer has previously received adjuvant therapy for treating breast cancer and experienced treatment failure. In some embodiments, the subject with HER2-expressing recurrent and/or metastatic breast cancer has experienced disease recurrence during the period of receiving adjuvant therapy for treating breast cancer. In some embodiments, the subject with HER2-expressing recurrent and/or metastatic breast cancer has experienced disease recurrence after receiving adjuvant therapy for treating breast cancer. In some embodiments, the subject with HER2-expressing recurrent and/or metastatic breast cancer has previously received adjuvant therapy for treating breast cancer and experienced disease recurrence within 6 months (e.g., 1, 2, 3, 4, 5, or 6 months) after the end of the treatment. In some embodiments, the subject with HER2-low expressing recurrent and/or metastatic breast cancer has previously received adjuvant therapy for treating breast cancer and experienced treatment failure. In some embodiments, the subject with HER2-low expressing recurrent and/or metastatic breast cancer has experienced disease recurrence during the period of receiving adjuvant therapy for treating breast cancer. In some embodiments, the subject with HER2-low expressing recurrent and/or metastatic breast cancer has experienced disease recurrence after receiving adjuvant therapy for treating breast cancer. In some embodiments, the subject with HER2-low expressing recurrent and/or metastatic breast cancer has previously received adjuvant therapy for treating breast cancer and experienced disease recurrence within 6 months (e.g., 1, 2, 3, 4, 5, or 6 months) after the end of the treatment.

In some embodiments, the subject with breast cancer has previously received first-line endocrine therapy for treating breast cancer (e.g., treatment failure or intolerance). In some embodiments, the subject with breast cancer has previously received the combined use of a CDK4/6 inhibitor and endocrine therapy for treating breast cancer and experienced treatment failure. In some embodiments, the subject with breast cancer has experienced disease recurrence during the period of receiving adjuvant endocrine therapy for treating breast cancer. In some embodiments, the subject with breast cancer has previously received adjuvant endocrine therapy for treating breast cancer and experienced disease recurrence within 12 months after the end of the treatment. In some embodiments, the subject with recurrent and/or metastatic breast cancer has previously received first-line endocrine therapy for treating breast cancer (e.g., treatment failure or intolerance). In some embodiments, the subject with recurrent and/or metastatic breast cancer has previously received the combined use of a CDK4/6 inhibitor and endocrine therapy for treating breast cancer and experienced treatment failure. In some embodiments, the subject with recurrent and/or metastatic breast cancer has experienced disease recurrence during the period of receiving adjuvant endocrine therapy for treating breast cancer. In some embodiments, the subject with recurrent and/or metastatic breast cancer has previously received adjuvant endocrine therapy for treating breast cancer and experienced disease recurrence within 12 months after the end of the treatment. In some embodiments, the subject with HER2-expressing recurrent and/or metastatic breast cancer has previously received the combined use of a CDK4/6 inhibitor and endocrine therapy for treating breast cancer and experienced treatment failure. In some embodiments, the subject with HER2-expressing recurrent and/or metastatic breast cancer has experienced disease recurrence during the period of receiving adjuvant endocrine therapy for treating breast cancer. In some embodiments, the subject with HER2-expressing recurrent and/or metastatic breast cancer has previously received adjuvant endocrine therapy for treating breast cancer and experienced disease recurrence within 12 months after the end of the treatment. In some embodiments, the subject with HER2-low expressing recurrent and/or metastatic breast cancer has previously received the combined use of a CDK4/6 inhibitor and endocrine therapy for treating breast cancer and experienced treatment failure. In some embodiments, the subject with HER2-low expressing recurrent and/or metastatic breast cancer has experienced disease recurrence during the period of receiving adjuvant endocrine therapy for treating breast cancer. In some embodiments, the subject with HER2-low expressing recurrent and/or metastatic breast cancer has previously received adjuvant endocrine therapy for treating breast cancer and experienced disease recurrence within 12 months after the end of the treatment.

In some embodiments, the subject with breast cancer has previously received adjuvant therapy or neoadjuvant therapy for treating breast cancer (e.g., treatment failure or intolerance). In some embodiments, the subject with breast cancer has previously received adjuvant therapy or neoadjuvant therapy for treating breast cancer and experienced treatment failure. In some embodiments, the subject with breast cancer has experienced disease recurrence during the period of receiving adjuvant therapy or neoadjuvant therapy for treating breast cancer. In some embodiments, the subject with breast cancer has experienced disease recurrence after receiving adjuvant therapy or neoadjuvant therapy for treating breast cancer. In some embodiments, the subject with breast cancer has previously received adjuvant therapy or neoadjuvant therapy for treating breast cancer and experienced disease recurrence within 12 months after the end of the treatment. In some embodiments, the subject with recurrent and/or metastatic breast cancer has previously received adjuvant therapy or neoadjuvant therapy for treating breast cancer and experienced treatment failure. In some embodiments, the subject with recurrent and/or metastatic breast cancer has experienced disease recurrence during the period of receiving adjuvant therapy or neoadjuvant therapy for treating breast cancer. In some embodiments, the subject with recurrent and/or metastatic breast cancer has experienced disease recurrence after receiving adjuvant therapy or neoadjuvant therapy for treating breast cancer. In some embodiments, the subject with recurrent and/or metastatic breast cancer has previously received adjuvant therapy or neoadjuvant therapy for treating breast cancer and experienced disease recurrence within 12 months after the end of the treatment. In some embodiments, the subject with HER2-expressing recurrent and/or metastatic breast cancer has previously received adjuvant therapy or neoadjuvant therapy for treating breast cancer and experienced treatment failure. In some embodiments, the subject with HER2-expressing recurrent and/or metastatic breast cancer has experienced disease recurrence during the period of receiving adjuvant therapy or neoadjuvant therapy for treating breast cancer. In some embodiments, the subject with HER2-expressing recurrent and/or metastatic breast cancer has experienced disease recurrence after receiving adjuvant therapy or neoadjuvant therapy for treating breast cancer. In some embodiments, the subject with HER2-expressing recurrent and/or metastatic breast cancer has previously received adjuvant therapy or neoadjuvant therapy for treating breast cancer and experienced disease recurrence within 12 months after the end of the treatment. In some embodiments, the subject with HER2-positive recurrent and/or metastatic breast cancer has previously received adjuvant therapy or neoadjuvant therapy for treating breast cancer and experienced treatment failure. In some embodiments, the subject with HER2-positive recurrent and/or metastatic breast cancer has experienced disease recurrence during the period of receiving adjuvant therapy or neoadjuvant therapy for treating breast cancer. In some embodiments, the subject with HER2-positive recurrent and/or metastatic breast cancer has experienced disease recurrence after receiving adjuvant therapy or neoadjuvant therapy for treating breast cancer. In some embodiments, the subject with HER2-positive recurrent and/or metastatic breast cancer has previously received adjuvant therapy or neoadjuvant therapy for treating breast cancer and experienced disease recurrence within 12 months after the end of the treatment.

In some embodiments, the subject with breast cancer has previously received the treatment with an anti-HER2 antibody (e.g., trastuzumab) and/or a taxane drug for treating breast cancer (e.g., treatment failure or intolerance). In some embodiments, the subject with recurrent and/or metastatic breast cancer has previously received the treatment with an anti-HER2 antibody (e.g., trastuzumab) and/or a taxane drug for treating breast cancer (e.g., treatment failure or intolerance). In some embodiments, the subject with HER2-expressing recurrent and/or metastatic breast cancer has previously received the treatment with an anti-HER2 antibody (e.g., trastuzumab) and/or a taxane drug for treating breast cancer (e.g., treatment failure or intolerance). In some embodiments, the subject with HER2-positive recurrent and/or metastatic breast cancer has previously received the treatment with an anti-HER2 antibody (e.g., trastuzumab) and/or a taxane drug for treating breast cancer (e.g., treatment failure or intolerance).

In some embodiments, the subject with breast cancer has previously received local radiation therapy for treating breast cancer. In some embodiments, the subject with breast cancer has previously received local radiation therapy for treating breast cancer, the local radiation therapy is given more than 2 weeks prior to the first administration of the anti-HER2 antibody-drug conjugate, and a lesion targeted by the anti-HER2 antibody-drug conjugate is not within a local radiation therapy area. In some embodiments, the subject with breast cancer has previously received local radiation therapy for treating breast cancer, the local radiation therapy is administered more than 2 weeks prior to the first administration of the anti-HER2 antibody-drug conjugate, and the subject has developed progressive disease. In some embodiments, the local radiation therapy is palliative radiation therapy or brain radiation therapy. In some embodiments, the subject with breast cancer is a subject with HER2-expressing recurrent and/or metastatic breast cancer who has received and then failed at least one line of systemic chemotherapy in the recurrent and/or metastatic stage. In some embodiments, the subject with breast cancer is a hormone receptor-positive subject with HER2-expressing recurrent and/or metastatic breast cancer who has received and then failed the treatment of a CDK4/6 inhibitor in combination with endocrine therapy in the recurrent and/or metastatic stage. In some embodiments, the subject with breast cancer is a subject with HER2-expressing recurrent and/or metastatic breast cancer who has received the treatment with trastuzumab and a taxane drug.

In some embodiments, the subject with breast cancer is a subject with HER2-positive recurrent and/or metastatic breast cancer who has received and then failed at least one line of systemic chemotherapy in the recurrent and/or metastatic stage. In some embodiments, the subject with breast cancer is a subject with HER2-positive recurrent and/or metastatic breast cancer who has received the treatment with trastuzumab and a taxane drug. In some embodiments, the subject with breast cancer is a subject with recurrent and/or metastatic breast cancer determined to have HER2 expression of 3+ by IHC, or determined to be positive for HER2 expression by ISH, or determined to have HER2 expression of 2+ by IHC and determined to be positive for HER2 expression by ISH, who has received and then failed at least one line of systemic chemotherapy in the recurrent and/or metastatic stage. In some embodiments, the subject with breast cancer is a subject with recurrent and/or metastatic breast cancer determined to have HER2 expression of 3+ by IHC, or determined to be positive for HER2 expression by ISH, or determined to have HER2 expression of 2+ by IHC and determined to be positive for HER2 expression by ISH, who has received the treatment with trastuzumab and a taxane drug.

In some embodiments, the subject with breast cancer is a subject with HER2-low expressing recurrent and/or metastatic breast cancer who has received and then failed at least one line of systemic chemotherapy in the recurrent and/or metastatic stage. In some embodiments, the subject with breast cancer is a hormone receptor-positive subject with HER2-low expressing recurrent and/or metastatic breast cancer who has received and then failed the treatment of a CDK4/6 inhibitor in combination with endocrine therapy in the recurrent and/or metastatic stage. In some embodiments, the subject with breast cancer is a subject with recurrent and/or metastatic breast cancer determined to have HER2 expression of 1+ by IHC, or determined to have HER2 expression of 2+ by IHC and determined to be negative for HER2 expression by ISH, who has received and then failed at least one line of systemic chemotherapy in the recurrent and/or metastatic stage. In some embodiments, the subject with breast cancer is a hormone receptor-positive subject with recurrent and/or metastatic breast cancer determined to have HER2 expression of 1+ by IHC, or determined to have HER2 expression of 2+ by IHC and determined to be negative for HER2 expression by ISH, who has received and then failed the treatment of a CDK4/6 inhibitor in combination with endocrine therapy in the recurrent and/or metastatic stage.

In some embodiments, the subject with breast cancer is a subject with HER2-low expressing locally advanced or metastatic breast cancer who has not received chemotherapy in the recurrent and/or metastatic stage. In some embodiments, the subject with breast cancer is a subject with HER2-low expressing unresectable locally advanced or metastatic breast cancer who has not received chemotherapy in the recurrent and/or metastatic stage. In some embodiments, the subject with breast cancer is a subject with locally advanced or metastatic breast cancer determined to have HER2 expression of 1+ by IHC, or determined to have HER2 expression of 2+ by IHC and determined to be negative for HER2 expression by ISH, who has not received chemotherapy in the recurrent and/or metastatic stage. In some embodiments, the subject with breast cancer is a subject with unresectable locally advanced or metastatic breast cancer determined to have HER2 expression of 1+ by IHC, or determined to have HER2 expression of 2+ by IHC and determined to be negative for HER2 expression by ISH, who has not received chemotherapy in the recurrent and/or metastatic stage.

In some embodiments, the subject with breast cancer is a subject with HR-positive and HER2-low expressing locally advanced or metastatic breast cancer who has not received chemotherapy in the recurrent and/or metastatic stage. In some embodiments, the subject with breast cancer is a subject with HR-positive and HER2-low expressing locally advanced or metastatic breast cancer who has received at least one line of endocrine therapy in the recurrent and/or metastatic stage and developed progressive disease, or who has developed progressive disease within 24 months after the start of adjuvant endocrine therapy. In some embodiments, the subject with breast cancer is a subject with HR-positive and HER2-low expressing unresectable locally advanced or metastatic breast cancer who has not received chemotherapy in the recurrent and/or metastatic stage, or who has received at least one line of endocrine therapy in the recurrent and/or metastatic stage and developed progressive disease, or who has developed progressive disease within 24 months after the start of adjuvant endocrine therapy.

In some embodiments, the subject with breast cancer is a subject with hormone receptor-negative or positive and HER2-positive recurrent or metastatic breast cancer. In some embodiments, the subject with breast cancer is a subject with recurrent or metastatic breast cancer who is (1) negative or positive for the hormone receptor, and (2) determined to have HER2 expression of 3+ by IHC, or determined to be positive for HER2 expression by ISH, or determined to have HER2 expression of 2+ by IHC and determined to be positive for HER2 expression by ISH.

### Anti-HER2 Antibody-Drug Conjugate

The anti-HER2 antibody-drug conjugate used in the present disclosure is formed by linking a drug-linker having a structure represented by formula Ia below to an HER2-targeting antigen-binding construct: 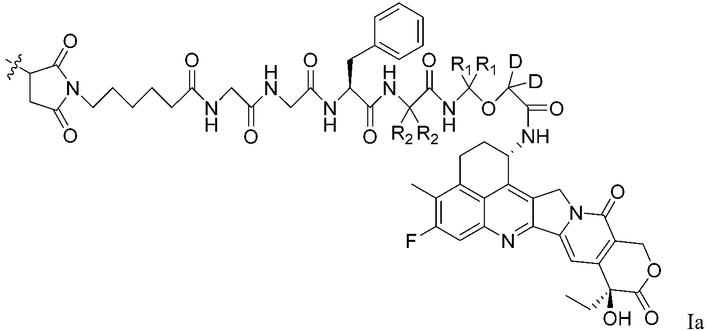
wherein R₁ and R₂ in formula Ia are each independently selected from hydrogen (H) and deuterium (D), and position 3 of -(succinimidyl-3-yl-N)- in formula Ia (i.e., the position linked by " 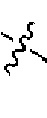 ") is linked to the HER2-targeting antigen-binding construct;
the HER2-targeting antigen-binding construct comprises a first antigen-binding fragment and a second antigen-binding fragment;
the first antigen-binding fragment comprises: a heavy chain CDR1 (HCDR1) comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, a light chain CDR1 (LCDR1) comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;
the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

In some embodiments, position 3 of -(succinimidyl-3-yl-N)- is linked to the HER2-targeting antigen-binding construct via a thioether bond. In some embodiments, R₁ and R₂ are hydrogen.

In some embodiments, in the anti-HER2 antibody-drug conjugate, the average number of linked drug-linkers per HER2-targeting antigen-binding construct is 2 to 8. In some embodiments, in the anti-HER2 antibody-drug conjugate, the average number of linked drug-linkers per HER2-targeting antigen-binding construct is 4 to 7. In some embodiments, in the anti-HER2 antibody-drug conjugate, the average number of linked drug-linkers per HER2-targeting antigen-binding construct is 5 to 6. In some embodiments, in the anti-HER2 antibody-drug conjugate, the average number of linked drug-linkers per HER2-targeting antigen-binding construct is 5.5 to 6. In some embodiments, in the anti-HER2 antibody-drug conjugate, the average number of linked drug-linkers per HER2-targeting antigen-binding construct is 5.8 to 6.

The anti-HER2 antibody-drug conjugate used in the present disclosure may also be represented by the structure represented by formula II below: 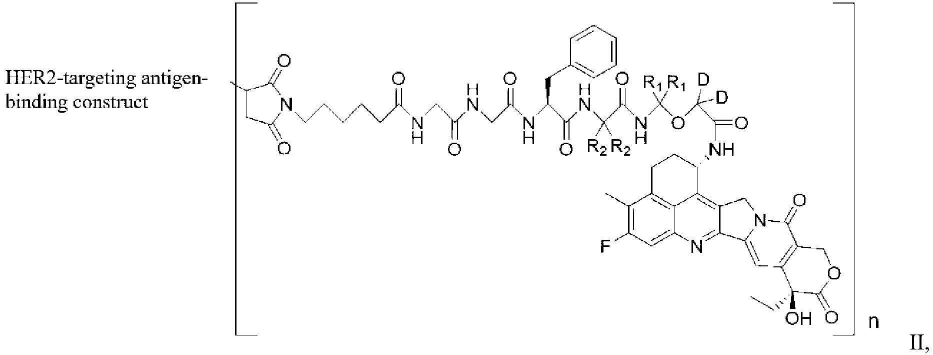 wherein R₁ and R₂ in formula II are each independently selected from hydrogen and deuterium. In some embodiments, the drug-linker (specifically, position 3 of -(succinimidyl-3-yl-N)-) is linked to the HER2-targeting antigen-binding construct via a thioether bond. In some embodiments, R₁ and R₂ are hydrogen. n has the same meaning as DAR and represents the average number of linked cytotoxic drugs per HER2-targeting antigen-binding construct. In some embodiments, n is 2 to 8. In some embodiments, n is 4 to 7. In some embodiments, n is 5 to 6. In some embodiments, n is 5.5 to 6. In some embodiments, n is 5.8 to 6.

**Table 1. CDR sequences of exemplary HER2-targeting antigen-binding construct**

| | | | |
|---|---|---|---|
| First antigen-binding fragment | HCDR1 | GFNIEDTYIH | SEQ ID NO: 1 |
| | HCDR2 | RIYPTNGYTRYADSVKG | SEQ ID NO: 2 |
| | HCDR3 | WGGDGFYAMDYW | SEQ ID NO: 3 |
| | LCDR1 | CRASQDVNTAVAW | SEQ ID NO: 4 |
| | LCDR2 | SASYLYS | SEQ ID NO: 5 |
| | LCDR3 | QQHYTTPPT | SEQ ID NO: 6 |
| Second antigen-binding fragment | HCDR1 | GFTFTDYTMD | SEQ ID NO: 9 |
| | HCDR2 | DVNPNSGGSIYNQRFKG | SEQ ID NO: 10 |
| | HCDR3 | NLGPSFYFDY | SEQ ID NO: 11 |
| | LCDR1 | KASQDVSIGVA | SEQ ID NO: 12 |
| | LCDR2 | SASYRYT | SEQ ID NO: 13 |
| | LCDR3 | QQYYIYPYT | SEQ ID NO: 14 |

It should be understood by those skilled in the art that, unless otherwise specified, the term "CDR" or "complementarity determining region" of a given antigen-binding fragment or a region thereof (e.g., a variable region) should be understood to encompass complementarity determining regions defined by any one of the known schemes. Although the CDRs claimed in the present disclosure are based on the sequences shown in Table 1 (one definition scheme), amino acid sequences corresponding to other CDR definition schemes (one of or a combination of several of the definition schemes well known in the art, such as AbM, CCG, Kabat, Chothia, IMGT, or Contact) should also fall within the protection scope of the present disclosure.

In some embodiments, the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8. In some embodiments, the first antigen-binding fragment comprises a heavy chain variable region having the amino acid sequence set forth in SEQ ID NO: 7 and a light chain variable region having the amino acid sequence set forth in SEQ ID NO: 8. In some specific embodiments, the amino acid sequence of the heavy chain variable region of the first antigen-binding fragment is set forth in SEQ ID NO: 7, and the amino acid sequence of the light chain variable region of the first antigen-binding fragment is set forth in SEQ ID NO: 8.

In some embodiments, the first antigen-binding fragment comprises a heavy chain variable region and a light chain variable region, wherein the heavy chain variable region comprises an HCDR1 having the amino acid sequence set forth in SEQ ID NO: 1, an HCDR2 having the amino acid sequence set forth in SEQ ID NO: 2, and an HCDR3 having the amino acid sequence set forth in SEQ ID NO: 3, and the light chain variable region comprises an LCDR1 having the amino acid sequence set forth in SEQ ID NO: 4, an LCDR2 having the amino acid sequence set forth in SEQ ID NO: 5, and an LCDR3 having the amino acid sequence set forth in SEQ ID NO: 6; and, the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 7, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 8.

EVQLVESGGGLVQPGGSLRLSCAASGFNIEDTYIHWVRQAPGKGLEWVARIYPTNGYTRYADSVKGRFTI
SADTSKNTAYLQMNSLRAEDTAVYYCSRWGGDGFYAMDYWGQGTLVTVSS (SEQ ID NO: 7);

DIQMTQSPSSLSASVGDRVTITCRASQDVNTAVAWYQQKPGKAPKLLIYSASYLYSGVPSRFSGSRSGTDF
TLTISSLQPEDFATYYCQQHYTTPPTFGQGTKVEIK (SEQ ID NO: 8).

In some embodiments, compared with the amino acid sequence set forth in SEQ ID NO: 7 or SEQ ID NO: 8, the differing amino acids in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity thereto are located in FR regions. In some embodiments, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16. In some embodiments, the second antigen-binding fragment comprises a heavy chain variable region having the amino acid sequence set forth in SEQ ID NO: 15 and a light chain variable region having the amino acid sequence set forth in SEQ ID NO: 16. In some specific embodiments, the amino acid sequence of the heavy chain variable region of the second antigen-binding fragment is set forth in SEQ ID NO: 15, and the amino acid sequence of the light chain variable region of the second antigen-binding fragment is set forth in SEQ ID NO: 16.

In some embodiments, the second antigen-binding fragment comprises a heavy chain variable region and a light chain variable region, wherein the heavy chain variable region comprises an HCDR1 having the amino acid sequence set forth in SEQ ID NO: 9, an HCDR2 having the amino acid sequence set forth in SEQ ID NO: 10, and an HCDR3 having the amino acid sequence set forth in SEQ ID NO: 11, and the light chain variable region comprises an LCDR1 having the amino acid sequence set forth in SEQ ID NO: 12, an LCDR2 having the amino acid sequence set forth in SEQ ID NO: 13, and an LCDR3 having the amino acid sequence set forth in SEQ ID NO: 14; and, the heavy chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 15, and the light chain variable region comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to SEQ ID NO: 16.


 (SEQ ID NO: 15);


 (SEQ ID NO: 16).

In some embodiments, compared with the amino acid sequence set forth in SEQ ID NO: 15 or SEQ ID NO: 16, the differing amino acids in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity thereto are located in the FR regions.

In some embodiments, the HER2-targeting antigen-binding construct may further comprise a constant region of an immunoglobulin, or a fragment, an analog, a variant, or a derivative of the constant region. In some embodiments, the constant region comprises a heavy chain constant region and a light chain constant region. In some embodiments, the heavy chain constant region is derived from a human immunoglobulin heavy chain, for example, a heavy chain of IgG1, IgG2, IgG3, and IgG4, or other types of immunoglobulins, preferably a heavy chain of IgG4. In some embodiments, the light chain constant region is derived from a human immunoglobulin light chain, for example, a κ light chain or a λ light chain of a human immunoglobulin. In some embodiments, the constant region may comprise any modification described in the text, for example, insertion, deletion, substitution, or chemical modification of amino acids. In some embodiments, the C-terminal lysine of the heavy chain constant region may be present or deleted, and the deletion of the C-terminal lysine of the heavy chain constant region generally occurs during recombinant expression. In some embodiments, the constant region comprises a mutation that alters the effector function. In some embodiments, any amino acid residue of the constant region may be substituted with an amino acid residue of any allotype.

In some embodiments, the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19. In some embodiments, the HER2-targeting antigen-binding construct comprises a first polypeptide chain having the amino acid sequence set forth in SEQ ID NO: 17, a second polypeptide chain having the amino acid sequence set forth in SEQ ID NO: 18, and a third polypeptide chain having the amino acid sequence set forth in SEQ ID NO: 19. In some specific embodiments, the HER2-targeting antigen-binding construct consists of three polypeptide chains, wherein the amino acid sequence of the first polypeptide chain is set forth in SEQ ID NO: 17, the amino acid sequence of the second polypeptide chain is set forth in SEQ ID NO: 18, and the amino acid sequence of the third polypeptide chain is set forth in SEQ ID NO: 19.

In some embodiments, the HER2-targeting antigen-binding construct comprises a first polypeptide chain, a second polypeptide chain, and a third polypeptide chain, wherein the first polypeptide chain comprises an HCDR1 having the amino acid sequence set forth in SEQ ID NO: 1, an HCDR2 having the amino acid sequence set forth in SEQ ID NO: 2, an HCDR3 having the amino acid sequence set forth in SEQ ID NO: 3, an LCDR1 having the amino acid sequence set forth in SEQ ID NO: 4, an LCDR2 having the amino acid sequence set forth in SEQ ID NO: 5, and an LCDR3 having the amino acid sequence set forth in SEQ ID NO: 6, the second polypeptide chain comprises an HCDR1 having the amino acid sequence set forth in SEQ ID NO: 9, an HCDR2 having the amino acid sequence set forth in SEQ ID NO: 10, and an HCDR3 having the amino acid sequence set forth in SEQ ID NO: 11, and the third polypeptide chain comprises an LCDR1 having the amino acid sequence set forth in SEQ ID NO: 12, an LCDR2 having the amino acid sequence set forth in SEQ ID NO: 13, and an LCDR3 having the amino acid sequence set forth in SEQ ID NO: 14; and, the first polypeptide chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, the second polypeptide chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and the third polypeptide chain comprises an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.

EVQLVESGGGLVQPGGSLRLSCAASGFNIEDTYIHWVRQAPGKGLEWVARIYPTNGYTRYADSVKGRFTI
SADTSKNTAYLQMNSLRAEDTAVYYCSRWGGDGFYAMDYWGQGTLVTVSSGGGGSGGGGSGGGGSGG
GGSDIQMTQSPSSLSASVGDRVTITCRASQDVNTAVAWYQQKPGKAPKLLIYSASYLYSGVPSRFSGSRSG
TDFTLTISSLQPEDFATYYCQQHYTTPPTFGQGTKVEIKGEPKSSDKTHTCPPCPAPELLGGPSVFLFPPKPK
DTLMISRTPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPCREEMTKNQVSLWCLVKGFYPSDIAVEWESNGQ
PENNYKTTPPVLDSDGSFFLYSKLTVDKSRWQQGNVFSCSVMHEALHNHYTQKSLSLSPGK (SEQ ID NO:
17);

EVQLVESGGGLVQPGGSLRLSCAASGFTFTDYTMDWVRQAPGKGLEWVADVNPNSGGSIYNQRFKGRF
TLSVDRSKNTLYLQMNSLRAEDTAVYYCARNLGPSFYFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPREP
QVCTLPPSREEMTKNQVSLSCAVKGFYPSDIAVEWESNGQPENNYKTTPPVLDSDGSFFLVSKLTVDKSR
WQQGNVFSCSVMHEALHNRFTQKSLSLSPGK (SEQ ID NO: 18);

DIQMTQSPSSLSASVGDRVTITCKASQDVSIGVAWYQQKPGKAPKLLIYSASYRYTGVPSRFSGSGSGTDF
TLTISSLQPEDFATYYCQQYYIYPYTFGQGTKVEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGE
C (SEQ ID NO: 19).

In some embodiments, the C-terminal lysine of the amino acid sequence set forth in SEQ ID NO: 17 is deleted, as set forth in SEQ ID NO: 20. In some embodiments, the C-terminal lysine of the amino acid sequence set forth in SEQ ID NO: 18 is deleted, as set forth in SEQ ID NO: 21. In some embodiments, the C-terminal lysine of each of the first polypeptide chain and the second polypeptide chain is deleted, the amino acid sequence of the first polypeptide chain is set forth in SEQ ID NO: 20, and the amino acid sequence of the second polypeptide chain is set forth in SEQ ID NO: 21.

EVQLVESGGGLVQPGGSLRLSCAASGFNIEDTYIHWVRQAPGKGLEWVARIYPTNGYTRYADSVKGRFTI
SADTSKNTAYLQMNSLRAEDTAVYYCSRWGGDGFYAMDYWGQGTLVTVSSGGGGSGGGGSGGGGSGG
GGSDIQMTQSPSSLSASVGDRVTITCRASQDVNTAVAWYQQKPGKAPKLLIYSASYLYSGVPSRFSGSRSG
TDFTLTISSLQPEDFATYYCQQHYTTPPTFGQGTKVEIKGEPKSSDKTHTCPPCPAPELLGGPSVFLFPPKPK
DTLMISRTPEVTCVVVDVSHEDPEVKFNWYVDGVEVHNAKTKPREEQYNSTYRVVSVLTVLHQDWLNG
KEYKCKVSNKALPAPIEKTISKAKGQPREPQVYTLPPCREEMTKNQVSLWCLVKGFYPSDIAVEWESNGQ
PENNYKTTPPVLDSDGSFFLYSKLTVDKSRWQQGNVFSCSVMHEALHNHYTQKSLSLSPG (SEQ ID NO:
20);

EVQLVESGGGLVQPGGSLRLSCAASGFTFTDYTMDWVRQAPGKGLEWVADVNPNSGGSIYNQRFKGRF
TLSVDRSKNTLYLQMNSLRAEDTAVYYCARNLGPSFYFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKPS
NTKVDKKVEPKSCDKTHTCPPCPAPELLGGPSVFLFPPKPKDTLMISRTPEVTCVVVDVSHEDPEVKFNW
YVDGVEVHNAKTKPREEQYNSTYRVVSVLTVLHQDWLNGKEYKCKVSNKALPAPIEKTISKAKGQPREP
QVCTLPPSREEMTKNQVSLSCAVKGFYPSDIAVEWESNGQPENNYKTTPPVLDSDGSFFLVSKLTVDKSR
WQQGNVFSCSVMHEALHNRFTQKSLSLSPG (SEQ ID NO: 21).

In some embodiments, compared with the amino acid sequence set forth in SEQ ID NO: 17, 18, or 19, the differing amino acids in an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity thereto are located in FR regions or constant regions.

In some other embodiments, the HER2-targeting antigen-binding construct is selected from Expi Her2-1, Expi Her2-3, Expi Her2-4, Expi Her2-5, 23C2 Her2-1, 23C2 Her2-3, 23C2 Her2-4, and 23C2 Her2-5 (see WO2021219046 or CN115279791A). In some other embodiments, the HER2-targeting antigen-binding construct is selected from zanidatamab (ZW25), KN026, MBS301, KM257, and BCD-147.

The antibody-drug conjugate preferably used in the present disclosure is formed by linking a drug-linker having a structure represented by formula Ia below to an HER2-targeting antigen-binding construct: 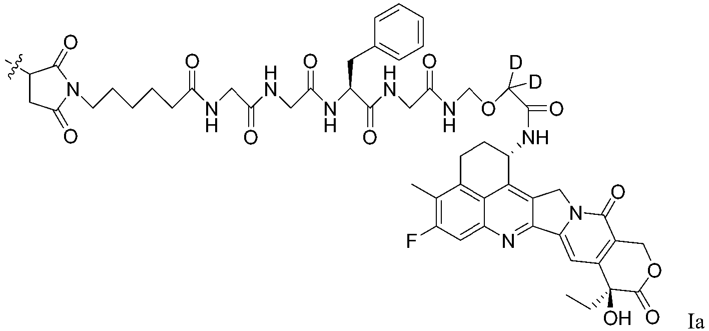
wherein position 3 of -(succinimidyl-3-yl-N)- in formula Ia is linked to the HER2-targeting antigen-binding construct;
the HER2-targeting antigen-binding construct consists of three polypeptide chains, wherein the amino acid sequence of the first polypeptide chain is set forth in SEQ ID NO: 17, the amino acid sequence of the second polypeptide chain is set forth in SEQ ID NO: 18, and the amino acid sequence of the third polypeptide chain is set forth in SEQ ID NO: 19; and
the average number of linked drug-linkers for one HER2-targeting antigen-binding construct is 5 to 6.

In some embodiments, position 3 of -(succinimidyl-3-yl-N)- in formula Ia is linked to the HER2-targeting antigen-binding construct via a thioether bond.

The antibody-drug conjugate preferably used in the present disclosure may also be represented by the structure represented by formula II below: 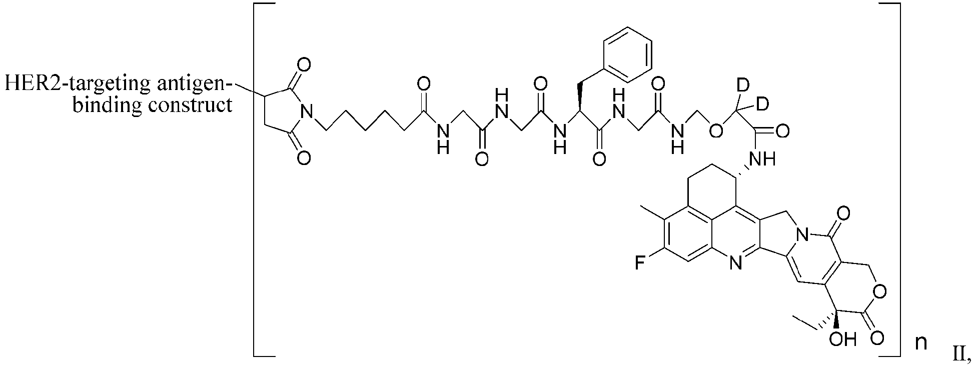
wherein n is 5 to 6;
the HER2-targeting antigen-binding construct consists of three polypeptide chains, wherein the amino acid sequence of the first polypeptide chain is set forth in SEQ ID NO: 17, the amino acid sequence of the second polypeptide chain is set forth in SEQ ID NO: 18, and the amino acid sequence of the third polypeptide chain is set forth in SEQ ID NO: 19. In some embodiments, the drug-linker is linked to the HER2-targeting antigen-binding construct via a thioether bond.
In some other specific embodiments, the anti-HER2 antibody-drug conjugate of the present disclosure is selected from Kadcyla^{®}, Enhertu^{®}, Aidixi^{®}, MRG002, ARX788, A166, SHR-A1811, BB-1701, SYD985, FS-1502, and BAT8001.
The anti-HER2 antibody-drug conjugate described in the present disclosure also includes isomers or pharmaceutically acceptable salts of the anti-HER2 antibody-drug conjugate, or solvates of the anti-HER2 antibody-drug conjugate, isomers thereof, or pharmaceutically acceptable salts thereof.

### Technical Effects

Administration of the anti-HER2 antibody-drug conjugate of the present disclosure achieves one or more of the following effects:
(1) providing benefit to a subject with breast cancer, wherein the breast cancer is preferably metastatic and/or recurrent breast cancer;
(2) exhibiting good safety;
(3) being well-tolerated in subjects.

### Definitions and Description

Unless otherwise stated, the following terms used in the present disclosure shall have the following meanings. A certain term, unless otherwise specifically defined, should not be considered uncertain or unclear, but interpreted according to its common meaning in the art. When referring to a trade name in the present disclosure, it is intended to refer to its corresponding commercial product or its active ingredient.

As used herein, the structure of "-(succinimidyl-3-yl-N)-" is as the formula below:

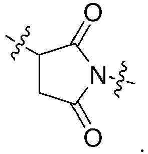

Unless otherwise stated, the absolute configuration of a stereogenic center is represented by a wedged solid bond ( 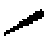 ) and a wedged dashed bond ( 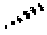 ).

Unless otherwise specified, when a certain group has a linkable site, the linkage between the site and another group may be represented by a wavy line ( 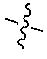 ).

The term "antigen-binding construct" refers to any agent capable of binding to an antigen, such as a polypeptide or a polypeptide complex. In some aspects, the antigen-binding construct is a polypeptide that specifically binds to a target antigen. The antigen-binding construct may be a monomer, a dimer, a polymer, a protein, a peptide, a protein or peptide complex, an antibody or an antigen-binding fragment thereof, and the like. The antigen-binding construct may be a monospecific, bispecific, or multispecific polypeptide construct. In some aspects, the antigen-binding construct may include, for example, one or more antigen-binding fragments (e.g., Fab or scFv) linked to one or more Fc.

The "antigen-binding fragment" of an antibody refers to one or more fragments of the antibody that retain the functionality of specifically binding to an antigen (e.g., HER2 protein). It has been demonstrated that the antigen-binding functionality of an antibody can be implemented by fragments of a full-length antibody. Examples encompassed within the term "antigen-binding fragment" of an antibody include: (i) a Fab fragment: a monovalent fragment consisting of VL, VH, CL, and CH1 domains; (ii) a F(ab')₂ fragment, a bivalent fragment comprising two Fab fragments linked by a disulfide bridge at the hinge region; (iii) an Fd fragment consisting of VH and CH1 domains; (iv) an Fv fragment consisting of VL and VH domains of a single arm of the antibody; (v) a dAb fragment consisting of VH domains (see Ward et al., Nature. 341:544-546 (1989)); and (vi) a nanobody, an antibody comprising a single variable domain and two constant domains. Furthermore, although the two domains VL and VH of the Fv fragment are encoded by different genes, the VH and VL can be joined via a linker by recombinant methods into a single protein chain in which the VL and VH are paired to form a monovalent molecule referred to as a single-chain Fv (scFv) (see, Bird et al., Science. 242:423-426 (1988); Huston et al., Proc. Natl. Acad. Sci. 85:5879-5883 (1988)). These single-chain antibodies are also encompassed within the term "antigen-binding fragment". These antibody fragments can be obtained using conventional techniques known to those skilled in the art, and the fragments can be subjected to functional screening using the same method as full-length antibodies.

The term "identity" is also referred to as consistency. The "percent identity (%)" of an amino acid sequence refers to the percentage of amino acid residues in a sequence to be aligned that are identical to those of a specific amino acid sequence as set forth herein when the sequence to be aligned is aligned with the specific amino acid sequence as set forth herein, with gaps introduced, if necessary, to achieve the maximum percent sequence identity and without considering any conservative replacements as part of the sequence identity. The alignment of amino acid sequences for identity can be performed in a variety of ways within the skill in the art, such as BLAST, BLAST-2, ALIGN, or Megalign (DNASTAR) software. Those skilled in the art can determine suitable parameters for aligning sequences, including any algorithms required to acquire the maximum alignment for the full length of sequences being compared.

The term "treating" refers to administering the compound described in the present disclosure to prevent, ameliorate, or eliminate a disease or one or more symptoms associated with the disease, and includes, but is not limited to:
(i) preventing the occurrence of a disease or disease state in a mammal, particularly when such a mammal is predisposed to the disease state but has not yet been diagnosed as having it;
(ii) inhibiting a disease or disease state, i.e., arresting its progression;
(iii) alleviating a disease or disease state, i.e., causing its regression; and
(iv) reducing any direct or indirect pathological consequences of a disease or disease state.

The term "therapeutically effective amount" refers to an amount of the compound of the present disclosure for (i) treating or preventing a specific disease, condition, or disorder, (ii) relieving, ameliorating, or eliminating one or more symptoms of a specific disease, condition, or disorder, or (iii) preventing or delaying the onset of one or more symptoms of the specific disease, condition, or disorder described herein. The amount of the compound of the present disclosure that constitutes a "therapeutically effective amount" may vary depending on factors such as the compound and its ability to elicit a desired response in an individual, the disease state and its severity, the mode of administration, and the age, sex, and body weight of the mammal to be treated.

The term "administer", "administration", or "administering" refers to physically introducing a therapeutic agent to a subject using any one of a variety of methods and delivery systems known to those skilled in the art.

Routes of administration for antibody-drug conjugates (e.g., anti-HER2 antibody-drug conjugates) include intravenous, intramuscular, intraperitoneal, spinal, or other parenteral routes of administration. As used herein, the term "parenteral administration" refers to modes of administration other than enteral administration, typically performed by injection, including, but not limited to, intravenous, intramuscular, intra-arterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural, and intrasternal injection and infusion, as well as *in vivo* electroporation. The administration may also be performed, for example, once, multiple times, and/or over one or more extended periods of time.

The term "pharmaceutically acceptable" is used herein for those compounds, materials, compositions, and/or dosage forms that are, within the scope of sound medical judgment, suitable for use in contact with the tissues of human beings and animals without excessive toxicity, irritation, allergic response, or other problems or complications, and commensurate with a reasonable benefit/risk ratio.

The term "pharmaceutically acceptable salt" refers to a salt of a compound (e.g., the antibody-drug conjugate of the present disclosure) that exhibits safety and efficacy when used in mammals and possesses the desired biological activity; for example, such a salt may be a metal salt, an ammonium salt, a salt formed with an organic base, a salt formed with an inorganic acid, a salt formed with an organic acid, a salt formed with a basic or acidic amino acid, or the like.

The term "excipient" refers to any ingredient other than the active ingredient (e.g., the antibody-drug conjugate of the present disclosure). The selection of the excipient will largely depend on factors such as the particular mode of administration, the effect of the excipient on solubility and stability, and the nature of the dosage form.

The term "solvate" refers to a substance formed by association of a compound with a solvent molecule.

In the present disclosure, "HER2-positive" breast cancer is not particularly limited as long as it is recognized by those skilled in the art as breast cancer with HER2 overexpression, and preferred examples include breast cancer determined to have HER2 expression of 3+ by immunohistochemistry (IHC) (i.e., determined as IHC 3+ by HER2 testing using IHC), or/and breast cancer determined to have HER2 expression of 2+ by IHC and determined to be positive for HER2 expression by in situ hybridization (ISH) (i.e., determined as IHC 2+ by HER2 testing using IHC and determined as ISH+ by HER2 testing using ISH). It should be noted that the in situ hybridization of the present disclosure includes, but is not limited to, fluorescence in situ hybridization (FISH) and dual-color in situ hybridization (DISH).

In the present disclosure, "HER2-low expressing" breast cancer is not particularly limited as long as it is recognized by those skilled in the art as breast cancer with low HER2 expression, and preferred examples include breast cancer determined to have HER2 expression of 2+ by IHC and determined to be negative for HER2 expression by ISH (i.e., determined as IHC 2+ by HER2 testing using IHC and determined as ISH- by HER2 testing using ISH), or/and breast cancer determined to have HER2 expression of 1+ by IHC (i.e., determined as IHC 1+ by HER2 testing using IHC).

Herein, the terms "subject", "patient", and "entity" can be used interchangeably. The "subject", "patient", or "entity" includes any human or non-human animal. The term "non-human animal" includes, but is not limited to, vertebrates such as non-human primates, sheep, dogs, and rodents such as mice, rats, and guinea pigs. In some embodiments, the subject, patient, or entity is a mammal. In some embodiments, the subject, patient, or entity is a mouse. In some embodiments, the subject, patient, or entity is a human.

As used herein, "combined use" or "use in combination" means that two or more active substances may be administered to a subject simultaneously or sequentially in any order as individual formulations. Alternatively, all of the active ingredients are formulated in a single formulation for simultaneous administration to a subject. Alternatively, a portion of the active ingredients is formulated into a single formulation, and the remaining portions of the active ingredients are each administered to a subject simultaneously or sequentially in any order as individual formulations.

The term "pharmaceutical composition" refers to a mixture composed of one or more active ingredients (e.g., the anti-HER2 antibody-drug conjugate of the present disclosure) and pharmaceutically acceptable excipients. The pharmaceutical composition is intended to facilitate the administration of the active ingredient(s) to a subject. Herein, the terms "pharmaceutical composition" and "formulation" have the same meaning and can be used interchangeably. As used herein, "treatment failure" is defined as the occurrence of progressive disease or disease recurrence during the treatment or after the last treatment.

As used herein, a certain "line of treatment" refers to a position in the sequence of different drugs or other therapies received by a patient. The term "first-line treatment" refers to a treatment with drugs that are the first or standard choice according to a patient's conditions. "Second-line treatment" and "third-line treatment" are administered after first-line treatment and second-line treatment, respectively. If a patient does not exhibit a positive clinical outcome to first-line treatment or second-line treatment, or exhibits a positive clinical response but subsequently develops progressive disease or experiences disease recurrence, or if the condition at that time develops resistance to a prior treatment that has elicited a positive clinical response, the patient will receive subsequent treatment regimens (e.g., second-line treatment or third-line treatment). Non-limiting examples of the clinical outcome include tumor response, overall survival, progression-free survival, disease-free survival, time to tumor recurrence, time to tumor progression, relative risks, toxicity, or side effects.

The word "comprise" and variations thereof such as "comprises" or "comprising" should be understood in an openended and non-exclusive sense, i.e., "including but not limited to".

Herein, unless otherwise specified clearly in the context, singular terms encompass plural referents, and vice versa. As used herein, "about" means being within an acceptable error range determined by those of ordinary skill in the art for a specific value, which will depend in part on how the value is measured or determined, i.e., the limitation by the measurement system. For example, "about" may mean being within 1× or more than 1× standard deviation, as practiced in the art. Alternatively, "about" may mean a range of up to +5%, for example, fluctuating within a specific numerical range given +2%, +1%, or +0.5%. Where a specific value is given in the present disclosure or in the claims, unless otherwise stated, "about" should be considered to mean being within an acceptable error range for that specific value. Herein, unless otherwise stated, all values for drug doses, timing, step parameters, or conditions are modified by "about" by default.

All patents, patent applications, and other identified publications are explicitly incorporated herein by reference for the purpose of description and disclosure. These publications are provided solely because they were disclosed prior to the filing date of the present disclosure. All statements as to the dates of these documents or descriptions as to the content of these documents are based on the information available to the applicant and do not constitute any admission as to the correctness of the dates or the content of these documents. Moreover, in any country or region, any reference to these publications herein shall not be construed as an admission that the publications form part of the commonly recognized knowledge in the art.

The present disclosure further provides the following specific embodiments, which, however, are not intended to limit the protection scope of the present disclosure:
Embodiment 1. A method for treating breast cancer in a subject, comprising administering to the subject an anti-HER2 antibody-drug conjugate, wherein the anti-HER2 antibody-drug conjugate is formed by linking a drug-linker having a structure represented by formula Ia below 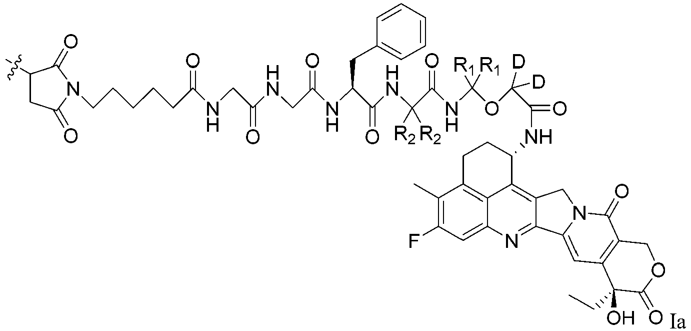
   to an HER2-targeting antigen-binding construct, wherein
   R₁ and R₂ in formula Ia are each independently selected from hydrogen (H) and deuterium (D), and position 3 of - (succinimidyl-3-yl-N)- is linked to the HER2-targeting antigen-binding construct;
   the HER2-targeting antigen-binding construct comprises a first antigen-binding fragment and a second antigen-binding fragment, wherein the first antigen-binding fragment comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, and the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.
Embodiment 2. The method according to embodiment 1, wherein an average number of linked drug-linkers per HER2-targeting antigen-binding construct is 4 to 7.
Embodiment 3. The method according to embodiment 2, wherein the average number of linked drug-linkers per HER2-targeting antigen-binding construct is 5 to 6.
Embodiment 4. The method according to embodiment 3, wherein the average number of linked drug-linkers per HER2-targeting antigen-binding construct is 5.5 to 6.
Embodiment 5. The method according to any one of embodiments 1-4, wherein
   (i) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7;
   (ii) the first antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iv) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15;
   (v) the second antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;
   (vi) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16; or
   (vii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8; and, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16.
Embodiment 6. The method according to any one of embodiments 1-5, wherein
   (i) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20;
   (ii) the HER2-targeting antigen-binding construct comprises a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21;
   (iii) the HER2-targeting antigen-binding construct comprises a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
   (iv) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;

   the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
   the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.
Embodiment 7. The method according to any one of embodiments 1-6, wherein the anti-HER2 antibody-drug conjugate is administered in a therapeutically effective amount.
Embodiment 8. The method according to any one of embodiments 1-7, wherein the anti-HER2 antibody-drug conjugate is administered at a dose of 1.5-9 mg/kg, 4.5-7.5 mg/kg, or 6-7.5 mg/kg each time; preferably, the anti-HER2 antibody-drug conjugate is administered at a dose of 6-7.5 mg/kg each time.
Embodiment 9. The method according to embodiment 8, wherein the anti-HER2 antibody-drug conjugate is administered at a dose of 1.5 mg/kg, 3 mg/kg, 4.5 mg/kg, 6 mg/kg, 7.5 mg/kg, or 9 mg/kg each time; preferably, the anti-HER2 antibody-drug conjugate is administered at a dose of 6 mg/kg or 7.5 mg/kg each time.
Embodiment 10. The method according to any one of embodiments 1-9, wherein the anti-HER2 antibody-drug conjugate is administered once every 1 week, every 2 weeks, every 3 weeks, or every 4 weeks; preferably, the anti-HER2 antibody-drug conjugate is administered once every 3 weeks.
Embodiment 11. The method according to any one of embodiments 1-10, wherein the anti-HER2 antibody-drug conjugate is formulated into a formulation for intravenous injection or infusion.
Embodiment 12. The method according to any one of embodiments 1-11, wherein the anti-HER2 antibody-drug conjugate is administered by intravenous infusion.
Embodiment 13. The method according to any one of embodiments 1-12, wherein the breast cancer is recurrent and/or metastatic breast cancer or locally advanced or metastatic breast cancer.
Embodiment 14. The method according to any one of embodiments 1-13, wherein the breast cancer is unresectable breast cancer.
Embodiment 15. The method according to any one of embodiments 1-14, wherein the breast cancer is HER2-expressing breast cancer.
Embodiment 16. The method according to any one of embodiments 1-15, wherein the breast cancer is HER2-positive breast cancer.
Embodiment 17. The method according to embodiment 16, wherein the HER2-positive breast cancer is breast cancer determined to have HER2 expression of 3+ by IHC.
Embodiment 18. The method according to embodiment 16, wherein the HER2-positive breast cancer is breast cancer determined to have HER2 expression of 2+ by IHC and determined to be positive for HER2 expression by ISH.
Embodiment 19. The method according to any one of embodiments 1-15, wherein the breast cancer is HER2-low expressing breast cancer.
Embodiment 20. The method according to embodiment 19, wherein the HER2-low expressing breast cancer is breast cancer determined to have HER2 expression of 2+ by IHC and determined to be negative for HER2 expression by ISH.
Embodiment 21. The method according to embodiment 19, wherein the HER2-low expressing breast cancer is breast cancer determined to have HER2 expression of 1+ by IHC.
Embodiment 22. The method according to any one of embodiments 1-21, wherein the breast cancer is hormone receptor-positive or hormone receptor-negative breast cancer.
Embodiment 23. The method according to any one of embodiments 1-22, wherein the breast cancer is hormone receptor-positive breast cancer.
Embodiment 24. The method according to embodiment 23, wherein the breast cancer is estrogen receptor- or/and progesterone receptor-positive breast cancer.
Embodiment 25. The use according to any one of embodiments 1-18, wherein the breast cancer is hormone receptor-negative or positive and HER2-positive recurrent or metastatic breast cancer.
Embodiment 26. The method according to any one of embodiments 1-25, wherein the subject with breast cancer is unsuitable for receiving surgery and/or radiation therapy.
Embodiment 27. The method according to any one of embodiments 1-26, wherein the subject with breast cancer has previously received at least one line of treatment for treating breast cancer.
Embodiment 28. The method according to any one of embodiments 1-27, wherein the subject with breast cancer is a subject with HER2-expressing recurrent and/or metastatic breast cancer who has received and then failed at least one line of systemic chemotherapy in the recurrent and/or metastatic stage;
   the subject with breast cancer is a hormone receptor-positive subject with HER2-expressing recurrent and/or metastatic breast cancer who has received and then failed the treatment of a CDK4/6 inhibitor in combination with endocrine therapy in the recurrent and/or metastatic stage;
   the subject with breast cancer is a subject with HER2-expressing recurrent and/or metastatic breast cancer who has received the treatment with trastuzumab and a taxane drug;
   the subject with breast cancer is a subject with HER2-low expressing locally advanced or metastatic breast cancer who has not received chemotherapy in the recurrent and/or metastatic stage; or
   the subject with breast cancer is a subject with HR-positive and HER2-low expressing locally advanced or metastatic breast cancer who has received at least one line of endocrine therapy in the recurrent and/or metastatic stage and developed progressive disease, or who has developed progressive disease within 24 months after the start of adjuvant endocrine therapy.
Embodiment 29. The method according to any one of claims 1-28, wherein the subject with breast cancer is a subject with HER2-positive recurrent and/or metastatic breast cancer who has received and then failed at least one line of systemic chemotherapy in the recurrent and/or metastatic stage;
   the subject with breast cancer is a subject with HER2-positive recurrent and/or metastatic breast cancer who has received the treatment with trastuzumab and a taxane drug;
   the subject with breast cancer is a subject with HER2-low expressing recurrent and/or metastatic breast cancer who has received and then failed at least one line of systemic chemotherapy in the recurrent and/or metastatic stage;
   the subject with breast cancer is a hormone receptor-positive subject with HER2-low expressing recurrent and/or metastatic breast cancer who has received and then failed the treatment of a CDK4/6 inhibitor in combination with endocrine therapy in the recurrent and/or metastatic stage;
   the subject with breast cancer is a subject with HER2-low expressing unresectable locally advanced or metastatic breast cancer who has not received chemotherapy in the recurrent and/or metastatic stage; or
   the subject with breast cancer is a subject with HR-positive and HER2-low expressing unresectable locally advanced or metastatic breast cancer who has received at least one line of endocrine therapy in the recurrent and/or metastatic stage and developed progressive disease, or who has developed progressive disease within 24 months after the start of adjuvant endocrine therapy.
Embodiment 30. The method according to any one of embodiments 1-29, wherein the anti-HER2 antibody-drug conjugate is for use in treating breast cancer in a subject in second-line treatment or a later line of treatment.
Embodiment 31. The method according to any one of embodiments 1-30, wherein the subject with breast cancer has previously received adjuvant therapy or neoadjuvant therapy for treating breast cancer.
Embodiment 32. The method according to any one of embodiments 1-31, wherein the subject with breast cancer has previously received local radiation therapy for treating breast cancer.
Embodiment 33. The method according to any one of embodiments 1-32, wherein the subject with breast cancer has previously received anti-HER2 antibody treatment, a taxane drug, a CDK4/6 inhibitor, endocrine therapy, and/or adjuvant endocrine therapy.
Embodiment 34. The method according to any one of embodiments 1-33, wherein the subject with breast cancer has not received chemotherapy in the recurrent and/or metastatic stage.
Embodiment 35. Use of the anti-HER2 antibody-drug conjugate for preparing a medicament for treating breast cancer in a subject, comprising administering to the subject the anti-HER2 antibody-drug conjugate, wherein the anti-HER2 antibody-drug conjugate is formed by linking a drug-linker having a structure represented by formula Ia below 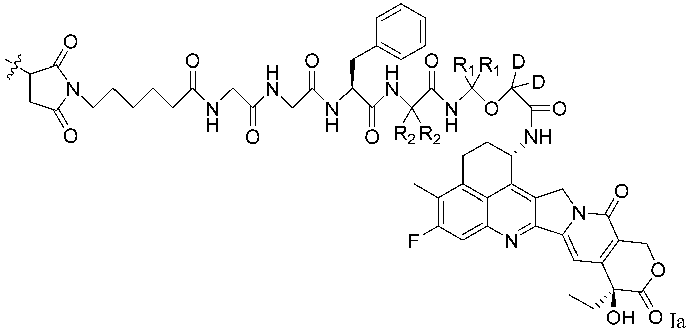
   to an HER2-targeting antigen-binding construct, wherein
   R₁ and R₂ in formula Ia are each independently selected from hydrogen (H) and deuterium (D), and position 3 of - (succinimidyl-3-yl-N)- is linked to the HER2-targeting antigen-binding construct;
   the HER2-targeting antigen-binding construct comprises a first antigen-binding fragment and a second antigen-binding fragment, wherein the first antigen-binding fragment comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6, and the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.
Embodiment 36. The use according to embodiment 35, wherein an average number of linked drug-linkers per HER2-targeting antigen-binding construct is 4 to 7.
Embodiment 37. The use according to embodiment 36, wherein the average number of linked drug-linkers per HER2-targeting antigen-binding construct is 5 to 6.
Embodiment 38. The use according to embodiment 37, wherein the average number of linked drug-linkers per HER2-targeting antigen-binding construct is 5.5 to 6.
Embodiment 39. The use according to any one of embodiments 35-38, wherein
   (i) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7;
   (ii) the first antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
   (iv) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15;
   (v) the second antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;
   (vi) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16; or
   (vii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8; and, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16.
Embodiment 40. The use according to any one of embodiments 35-39, wherein
   (i) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20;
   (ii) the HER2-targeting antigen-binding construct comprises a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21;
   (iii) the HER2-targeting antigen-binding construct comprises a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
   (iv) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;

   the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19;
   the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
   the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.
Embodiment 41. The use according to any one of embodiments 35-40, wherein the anti-HER2 antibody-drug conjugate is administered in a therapeutically effective amount.
Embodiment 42. The use according to any one of embodiments 35-41, wherein the anti-HER2 antibody-drug conjugate is administered at a dose of 1.5-9 mg/kg, 4.5-7.5 mg/kg, or 6-7.5 mg/kg each time; preferably, the anti-HER2 antibody-drug conjugate is administered at a dose of 6-7.5 mg/kg each time.
Embodiment 43. The use according to embodiment 42, wherein the anti-HER2 antibody-drug conjugate is administered at a dose of 1.5 mg/kg, 3 mg/kg, 4.5 mg/kg, 6 mg/kg, 7.5 mg/kg, or 9 mg/kg each time; preferably, the anti-HER2 antibody-drug conjugate is administered at a dose of 6 mg/kg or 7.5 mg/kg each time.
Embodiment 44. The use according to any one of embodiments 35-43, wherein the anti-HER2 antibody-drug conjugate is administered once every 1 week, every 2 weeks, every 3 weeks, or every 4 weeks; preferably, the anti-HER2 antibody-drug conjugate is administered once every 3 weeks.
Embodiment 45. The use according to any one of embodiments 35-44, wherein the anti-HER2 antibody-drug conjugate is formulated into a formulation for intravenous injection or infusion.
Embodiment 46. The use according to any one of embodiments 35-45, wherein the anti-HER2 antibody-drug conjugate is administered by intravenous infusion.
Embodiment 47. The use according to any one of embodiments 35-46, wherein the breast cancer is recurrent and/or metastatic breast cancer or locally advanced or metastatic breast cancer.
Embodiment 48. The use according to any one of embodiments 35-47, wherein the breast cancer is unresectable breast cancer.
Embodiment 49. The use according to any one of embodiments 35-48, wherein the breast cancer is HER2-expressing breast cancer.
Embodiment 50. The use according to any one of embodiments 35-49, wherein the breast cancer is HER2-positive breast cancer.
Embodiment 51. The use according to embodiment 50, wherein the HER2-positive breast cancer is breast cancer determined to have HER2 expression of 3+ by IHC.
Embodiment 52. The use according to embodiment 50, wherein the HER2-positive breast cancer is breast cancer determined to have HER2 expression of 2+ by IHC and determined to be positive for HER2 expression by ISH.
Embodiment 53. The use according to any one of embodiments 35-49, wherein the breast cancer is HER2-low expressing breast cancer.
Embodiment 54. The use according to embodiment 53, wherein the HER2-low expressing breast cancer is breast cancer determined to have HER2 expression of 2+ by IHC and determined to be negative for HER2 expression by ISH.
Embodiment 55. The use according to embodiment 53, wherein the HER2-low expressing breast cancer is breast cancer determined to have HER2 expression of 1+ by IHC.
Embodiment 56. The use according to any one of embodiments 35-55, wherein the breast cancer is hormone receptor-positive or hormone receptor-negative breast cancer.
Embodiment 57. The use according to any one of embodiments 35-56, wherein the breast cancer is hormone receptor-positive breast cancer.
Embodiment 58. The use according to embodiment 57, wherein the breast cancer is estrogen receptor- or/and progesterone receptor-positive breast cancer.
Embodiment 59. The use according to any one of embodiments 35-52, wherein the breast cancer is hormone receptor-negative or positive and HER2-positive recurrent or metastatic breast cancer.
Embodiment 60. The use according to any one of embodiments 35-59, wherein the subject with breast cancer is unsuitable for receiving surgery and/or radiation therapy.
Embodiment 61. The use according to any one of embodiments 35-60, wherein the subject with breast cancer has previously received at least one line of treatment for treating breast cancer.
Embodiment 62. The use according to any one of embodiments 35-61, wherein the subject with breast cancer is a subject with HER2-expressing recurrent and/or metastatic breast cancer who has received and then failed at least one line of systemic chemotherapy in the recurrent and/or metastatic stage;
   the subject with breast cancer is a hormone receptor-positive subject with HER2-expressing recurrent and/or metastatic breast cancer who has received and then failed the treatment of a CDK4/6 inhibitor in combination with endocrine therapy in the recurrent and/or metastatic stage;
   the subject with breast cancer is a subject with HER2-expressing recurrent and/or metastatic breast cancer who has received the treatment with trastuzumab and a taxane drug;
   the subject with breast cancer is a subject with HER2-low expressing locally advanced or metastatic breast cancer who has not received chemotherapy in the recurrent and/or metastatic stage; or
   the subject with breast cancer is a subject with HR-positive and HER2-low expressing locally advanced or metastatic breast cancer who has received at least one line of endocrine therapy in the recurrent and/or metastatic stage and developed progressive disease, or who has developed progressive disease within 24 months after the start of adjuvant endocrine therapy.
Embodiment 63. The use according to any one of claims 35-62, wherein the subject with breast cancer is a subject with HER2-positive recurrent and/or metastatic breast cancer who has received and then failed at least one line of systemic chemotherapy in the recurrent and/or metastatic stage;
   the subject with breast cancer is a subject with HER2-positive recurrent and/or metastatic breast cancer who has received the treatment with trastuzumab and a taxane drug;
   the subject with breast cancer is a subject with HER2-low expressing recurrent and/or metastatic breast cancer who has received and then failed at least one line of systemic chemotherapy in the recurrent and/or metastatic stage;
   the subject with breast cancer is a hormone receptor-positive subject with HER2-low expressing recurrent and/or metastatic breast cancer who has received and then failed the treatment of a CDK4/6 inhibitor in combination with endocrine therapy in the recurrent and/or metastatic stage;
   the subject with breast cancer is a subject with HER2-low expressing unresectable locally advanced or metastatic breast cancer who has not received chemotherapy in the recurrent and/or metastatic stage; or
   the subject with breast cancer is a subject with HR-positive and HER2-low expressing unresectable locally advanced or metastatic breast cancer who has received at least one line of endocrine therapy in the recurrent and/or metastatic stage and developed progressive disease, or who has developed progressive disease within 24 months after the start of adjuvant endocrine therapy.
Embodiment 64. The use according to any one of embodiments 35-63, wherein the anti-HER2 antibody-drug conjugate is for use in treating breast cancer in a subject in second-line treatment or a later line of treatment.
Embodiment 65. The use according to any one of embodiments 35-64, wherein the subject with breast cancer has previously received adjuvant therapy or neoadjuvant therapy for treating breast cancer.
Embodiment 66. The use according to any one of embodiments 35-65, wherein the subject with breast cancer has previously received local radiation therapy for treating breast cancer.
Embodiment 67. The use according to any one of embodiments 35-66, wherein the subject with breast cancer has previously received anti-HER2 antibody treatment, a taxane drug, a CDK4/6 inhibitor, endocrine therapy, and/or adjuvant endocrine therapy.
Embodiment 68. The use according to any one of embodiments 35-67, wherein the subject with breast cancer has not received chemotherapy in the recurrent and/or metastatic stage.

### Examples

For clarity, the present disclosure is further described with the following examples, which are, however, not intended to limit the scope of the present disclosure.

The content of the patent application WO2022033578 or CN115702008A is incorporated herein by reference in its entirety. In the following examples, the anti-HER2 antibody-drug conjugate is prepared according to the preparation method described in WO2022033578 to obtain the anti-HER2 antibody-drug conjugate represented by the formula below (hereinafter referred to as ADC1): 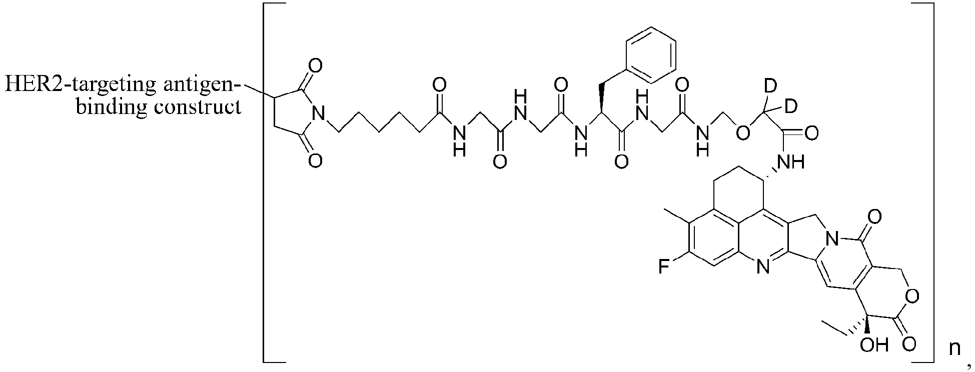 wherein the drug-linker is linked to the HER2-targeting antigen-binding construct via a thioether bond, and n is 5 to 6. Briefly, the nucleic acid sequences encoding the three polypeptide chains of the HER2-targeting antigen-binding construct (with amino acid sequences set forth in SEQ ID NOs: 17, 18, and 19, respectively) are each cloned into a pcDNA3.1 expression vector, and the vectors were co-transfected into FUT8-knockout CHO-S cells for expression. The HER2-targeting antigen-binding construct is then prepared after purification via Protein A. The HER2-targeting antigen-binding construct is treated with tris(2-carboxyethyl)phosphine hydrochloride and subsequently reacted with a linker-payload selected from the structure represented by the formula below to finally obtain ADC1:

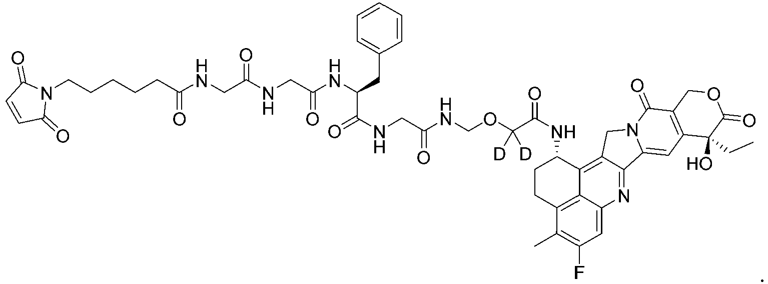

### Example 1: Phase Ib Clinical Trial of HER2-Expressing Breast Cancer

### 1. Study objectives:

### 1.1 Primary objective:

To evaluate the efficacy of ADC1 in subjects with HER2-expressing recurrent and/or metastatic breast cancer.

### 1.2 Secondary objectives:

(1) To evaluate the safety of ADC in subjects with HER2-expressing recurrent and/or metastatic breast cancer;
(2) To evaluate the pharmacokinetic (PK) profiles and immunogenicity of ADC1 in subjects with HER2-expressing recurrent and/or metastatic breast cancer.

### 2. Inclusion criteria

Subjects who meet all of the following inclusion criteria can be enrolled in this trial:
(1) Subjects voluntarily participated in this study and signed the informed consent form;
(2) Age: 18-75 years old (at the time of signing the informed consent form); ECOG score: ≤ 1; expected survival time of more than 3 months;
(3) Patients who are pathologically diagnosed with HER2-expressing breast cancer, with evidence of local lesion recurrence or distant metastasis, and who are unsuitable for receiving surgery or radiation therapy for curative purposes;
   a) HER2 expression is defined as: 1+, 2+, or 3+ as determined by standard immunohistochemistry (IHC);
   b) Low HER2 expression (cohort 1 subjects) is defined as: 1+ or 2+ as determined by standard immunohistochemistry (IHC); if the test shows 2+, negativity must be confirmed by in situ hybridization (ISH);
   c) HER2-positive (cohort 2 subjects) is defined as: 3+ or 2+ as determined by standard immunohistochemistry (IHC); if the test shows 2+, positivity must be confirmed by in situ hybridization (ISH), or positivity is determined by ISH alone;
(4) Prior treatment for cohort 1 subjects (low HER2 expression) must meet the following requirements:
   a) Subjects have received and then failed at least one line of systemic chemotherapy in the recurrent and/or metastatic stage; those who have experienced recurrence during adjuvant chemotherapy or within 6 months after the end of adjuvant chemotherapy are considered to have failed first-line chemotherapy;
   b) Hormone receptor-positive (HR-positive) subjects are required to have received and then failed the treatment of a CDK4/6 inhibitor in combination with endocrine therapy in the recurrent and/or metastatic stage; those who have experienced recurrence during adjuvant endocrine therapy or within 12 months after the end of adjuvant endocrine therapy are considered to have failed first-line endocrine therapy (HR-positive includes ER-positive and/or PR-positive, defined as: the proportion of positively stained tumor cells to all tumor cells ≥ 1%);
(5) Prior treatment for cohort 2 subjects (HER2-positive) must meet the following requirements:
   a) At least one line of systemic treatment in the recurrent and/or metastatic stage has been received and then has failed; subjects who have experienced recurrence during (neo)adjuvant therapy or within 12 months after the end of (neo)adjuvant therapy are considered to have failed first-line treatment;
   b) Subjects have received the treatment with trastuzumab and a taxane drug in any prior stage of the disease;
(6) Subjects have developed progressive disease or intolerance during or after the completion of the most recent treatment before enrollment;
(7) Having at least 1 measurable lesion according to RECIST 1.1 criteria;
(8) Good function of major organs, meeting the criteria for blood routine examination, biochemical examination, coagulation function examination, and cardiac color ultrasound assessment;
(9) Female subjects of childbearing age should agree to take necessary contraceptive measures (such as intrauterine devices, contraceptives, or condoms) during the study and for 6 months after the study; the serum pregnancy test results should be negative within 7 days before enrollment, and the subjects must be in the non-lactation period; male subjects should agree to take necessary contraceptive measures during the study and for 6 months after the study.

### 3. Test drug

ADC1 for injection (specification: 100 mg/vial), developed and provided by Chia Tai Tianqing Pharmaceutical Group Shunxin Pharmaceutical Co., Ltd.

### 4. Treatment regimen

Subjects in cohort 1 were randomly assigned to 2 dose groups (planned administration doses of 6.0 mg/kg and 7.5 mg/kg, respectively) according to the enrollment order. Subjects in cohort 2 were also divided into 2 dose groups (administration doses of 6.0 mg/kg and 7.5 mg/kg, respectively), and it was planned that about the first 50 subjects in cohort 2 received an administration dose of 6.0 mg/kg, and subsequently enrolled subjects received an administration dose of 7.5 mg/kg. The administration regimens for both cohorts could be adjusted according to the severity of the disease, the response of the disease, any treatment-related toxicity, and the age and health status of the patients.

ADC1 was administered via intravenous infusion once every 3 weeks, with 21 days as one treatment cycle, until progressive disease was developed or until the investigator determined that the subject was unsuitable for continued treatment.

### 5. Evaluation criteria

Efficacy evaluation: the disease status was determined using the RECIST 1.1 criteria.

Safety evaluation: the severity of adverse events was determined using the NCI-CTC AE 5.0 criteria.

### 6. Endpoint indicators

Objective response rate (ORR), disease control rate (DCR), duration of response (DOR), progression-free survival (PFS), overall survival (OS), clinical benefit rate (CBR), etc.;
Incidence and severity of adverse events (AEs), serious adverse events (SAEs), etc.;
Pharmacokinetic parameters, immunogenicity parameters (e.g., incidence of anti-drug antibodies), etc.

### 7. Results

### 7.1 Cohort 1

From November 21, 2023, to March 28, 2024, a total of 73 subjects in cohort 1 received at least one dose of ADC1 at 6.0 mg/kg (n = 37) or ADC1 at 7.5 mg/kg (n = 36). All subjects were female with a median age of 53 years. Among them, 48 subjects were determined as IHC 1+ by HER2 testing via IHC, and the other subjects were determined as IHC 2+ by HER2 testing via IHC and ISH- by HER2 testing via ISH. 73 subjects had received chemotherapy in the metastatic stage, and all HR-positive subjects (n = 50) had previously received CDK4/6 inhibitor treatment. In cohort 1, the median number of prior lines of treatment received by the subjects in the metastatic stage was 4 (range: 1-10), and the median number of prior lines of chemotherapy was 2 (range: 1-5). 12.3% (n = 9) of the subjects had previously received ADC treatment. Among them, 5 subjects had previously received anti-HER2 ADC treatment, and 5 subjects had previously received TROP2-targeting ADC treatment.

Based on the data as of August 1, 2024, the ORR of subjects in cohort 1 was 50.7% (37/73), wherein the ORR of the 7.5 mg/kg administration dose group (58.3%, 21/36) was superior to that of the 6.0 mg/kg administration dose group (43.2%, 16/37); the ORRs for HR-positive and HR-negative subjects were 50.0% (25/50) and 52.2% (12/23), respectively. The ORR for subjects in cohort 1 who had previously received ADC treatment was 44.4% (4/9). Surprisingly, no interstitial lung disease (ILD) was reported in the subjects of cohort 1, indicating good safety. Exemplary results are as follows:

| | Subject 1 | Subject 2 |
|---|---|---|
| Diagnosis | Invasive carcinoma of the right breast | Left breast cancer, ER-positive, PR-positive, HER2 IHC 2+ and ISH- |
| Prior treatment history | (1) 2 cycles of chemotherapy with docetaxel injection (130 mg, q3w); | (1) Modified radical mastectomy for left-breast cancer was performed, followed by 8 cycles of adjuvant chemotherapy with epirubicin and cyclophosphamide in combination with docetaxel (EC-T); |
| | (2) 6 cycles of chemotherapy with docetaxel injection (100 mg, q3w) in combination with epirubicin hydrochloride for injection (100 mg, q3w); | |
| | | (2) 25 sessions of radiation therapy were performed; |
| | 1 year of treatment with letrozole tablets (2.5 mg/time, once daily, oral administration) in combination with goserelin acetate sustained-release depot; | |
| | | (3) Treatment with letrozole was adopted, followed by development of progressive disease (PD); |
| | (3) 673 days of treatment with birociclib tablets (360 mg/time, twice daily, oral administration); | (4) First-line treatment with palbociclib in combination with exemestane was adopted, followed by development of PD; |
| | (4) 59 days of treatment with oral taragarestrant (200 mg/time, once daily, oral administration); | |
| | | (5) Second-line treatment with capecitabine, followed by development of PD; |
| | (5) Liver needle biopsy was performed, and pathological results showed poorly differentiated carcinoma visible (in the liver), with ER-positive cells accounting for about 30%, PR-negative, HER2 ICH 1+; | (6) 6 cycles of chemotherapy with gemcitabine in combination with eribulin were adopted, resulting in shrinkage of liver metastases; |
| | | (7) Treatment with fulvestrant was adopted, resulting in enlargement of liver metastases; |
| | (6) 3 cycles of treatment with disitamab vedotin; | (8) 3 cycles of chemotherapy with gemcitabine in combination with eribulin were adopted, |
| | (7) Oral palbociclib and megestrol acetate; | followed by development of PD. |
| | (8) CT suggested progressive disease. | |
| Treatment regimen | ADC1 at 7.5 mg/kg, q3w | ADC1 at 7.5 mg/kg, q3w for the first treatment cycle, followed by ADC 1 at 6.0 mg/kg, q3w for subsequent treatment cycles |
| Treatment results and evaluation | Screening period: target lesion: 27 mm; | Screening period: target lesion: 58 mm; |
| | 2 cycles of treatment: target lesion: 15 mm; | 2 cycles of treatment: target lesion: 39 mm; |
| | 4 cycles of treatment: target lesion: 14 mm; | 4 cycles of treatment: target lesion: 34 mm; |
| | 6 cycles of treatment: target lesion: 7 mm; | 6 cycles of treatment: target lesion: 28 mm; |
| | 8 cycles of treatment: target lesion: 7 mm; | 8 cycles of treatment: target lesion: 27 mm; |
| | The best response for patient was PR according to the Response Evaluation Criteria. | 10 cycles of treatment: target lesion: 28 mm; |
| | | The best response for patient was PR according to the Response Evaluation Criteria. |

### 7.2 Cohort 2

Exemplary results are as follows:

| | Subject 3 | Subject 4 | Subject 5 |
|---|---|---|---|
| Diagnosis | Breast cancer, ER-positive, PR-positive, HER2 ICH 2+ and ISH+ | Invasive carcinoma of the left breast, ER-positive, PR-positive, HER2 ICH 3+ | Invasive ductal carcinoma of the left breast, ER-negative, PR-negative, HER2 ICH 3+ |
| Prior treatment history | (1) 1 month of adjuvant chemotherapy with cyclophosphamide injection in combination with epirubicin hydrochloride injection; | (1) 6 cycles of treatment with docetaxel and pirarubicin in combination with cyclophosphamide (TAC) regimen were adopted, in combination with targeted therapy with pertuzumab and trastuzumab; | (1) 13 cycles of treatment with QL1701 (Anqutuo^{®}) or Herceptin in combination with docetaxel were adopted; |
| | (2) 2 months of treatment with paclitaxel injection; | | |
| | (3) First-line treatment with pertuzumab biosimilar; | | (2) 5 cycles of treatment with QL1701 or Herceptin were adopted; |
| | (4) Radiation therapy for the entire chest wall + left supraclavicular area was performed at a dose of 24 Gy/12f; | (2) 6 cycles of treatment with paclitaxel in combination with trastuzumab (TH) regimen were adopted; | (3) Treatment with trastuzumab was adopted, followed by development of PD; |
| | (5) Subtotal thyroidectomy of the left side was performed; | | |
| | | (3) Treatment with pyrotinib in combination with capecitabine was adopted; | (4) 12 cycles of treatment with tucatinib or placebo in combination with trastuzumab emtansine, followed by development of PD. |
| | (6) Development of progressive disease, bone metastasis; | | |
| | (7) 10 sessions of first-line palliative radiation therapy were performed at a dose of 30 Gy/10f; Pain was relieved after radiation therapy, and endocrine therapy with letrozole in combination with goserelin acetate was adopted, during which no abnormality was found in regular re-examinations; | (4) CT showed multiple metastases; | |
| | | (5) 1 cycle of treatment with inetetamab (400 mg) in combination with a taxane drug regimen was adopted; | |
| | | (6) 1 cycle of treatment with inetetamab and a taxane in combination with cisplatin regimen was adopted; | |
| | (8) Development of progressive disease; multiple solid space-occupying lesions in the liver were found by color ultrasound; | | |
| | (9) Second-line treatment with trastuzumab and docetaxel in combination with capecitabine was adopted, followed by treatment with trastuzumab; | | |
| | (10) Development of progressive disease; CT showed enlarged mediastinal lymph nodes. | | |
| Treatment regimen | ADC1 at 6.0 mg/kg, q3w | ADC1 at 7.5 mg/kg, q3w | ADC1 at 6.0 mg/kg, q3w |
| Treatment results and evaluation | Screening period: target lesion: 63 mm | Screening period: neck nodule 27 mm, left chest and abdominal subcutaneous nodule 71 mm, left axillary lymph node 35 mm, totaling 133 mm; | Screening period: target lesion: 67 mm; |
| | 6 cycles of treatment: target lesion: 28 mm; | | |
| | | | 2 cycles of treatment: target lesion: 50 mm; |
| | 12 cycles of treatment: target lesion: 26 mm; | | |
| | | | 4 cycles of treatment: target lesion: 30 mm; |
| | 18 cycles of treatment: target lesion: 21 mm; | | |
| | | 2 cycles of treatment: neck nodule 27 mm, left chest and abdominal subcutaneous nodule 58 mm, left axillary lymph node 20 mm, totaling 105 mm; | 6 cycles of treatment: target lesion: 0 mm; |
| | 24 cycles of treatment: target lesion: 21 mm; | | |
| | | | 8 cycles of treatment: target lesion: 0 mm; |
| | 30 cycles of treatment: target lesion: 21 mm; | | |
| | | | 10 cycles of treatment: target lesion: 0 mm; |
| | 42 cycles of treatment: target lesion: 21 mm; | | |
| | | 4 cycles of treatment: neck nodule 24 mm, left chest and abdominal subcutaneous nodule 22 mm, left axillary lymph node 18 mm, totaling 64 mm; | 12 cycles of treatment: target lesion: 0 mm; |
| | The best response for patient was PR according to the Response Evaluation Criteria. | | |
| | | | The best response for patient was PR according to the Response Evaluation Criteria, with the target lesion reaching CR. |
| . | | The best response for patient was PR according to the Response Evaluation Criteria. | |

### Example 2: Phase III Clinical Trial of HER2-Expressing Breast Cancer

### 1. Study objectives:

### 1.3 Primary objective:

To demonstrate that ADC1 can significantly prolong the progression-free survival (PFS) of subjects with HR-positive and HER2-low expressing recurrent and/or metastatic breast cancer compared with the investigator's choice of chemotherapy in the subjects.

### 1.4 Secondary objectives:

(1) To evaluate the progression-free survival (PFS) of subjects with HER2-low expressing recurrent and/or metastatic breast cancer by ADC1 compared with the investigator's choice of chemotherapy;
(2) To evaluate the overall survival (OS), objective response rate (ORR), duration of response (DOR), and clinical benefit rate (CBR) of subjects with HR-positive and HER2-low expressing recurrent and/or metastatic breast cancer by ADC1 compared with the investigator's choice of chemotherapy;
(3) To evaluate the overall survival (OS), objective response rate (ORR), duration of response (DOR), and clinical benefit rate (CBR) of subjects with HER2-low expressing recurrent and/or metastatic breast cancer by ADC1 compared with the investigator's choice of chemotherapy;
(4) To evaluate the safety of ADC1 compared with the investigator's choice of chemotherapy in subjects with HER2-low expressing recurrent and/or metastatic breast cancer, including: incidence and severity of adverse events (AEs), laboratory test value abnormalities, and serious adverse events (SAEs);
(5) To evaluate the pharmacokinetic (PK) profiles and immunogenicity of ADC1 in subjects with HER2-low expressing recurrent and/or metastatic breast cancer.

### 2. Inclusion criteria

Subjects who meet all of the following inclusion criteria can be enrolled in this trial:
(1) Subjects voluntarily participated in this study, signed the informed consent form, and demonstrated good compliance;
(2) Age: 18-75 years old (at the time of signing the informed consent form); ECOG score: 0 to 1; expected survival time of more than 3 months;
(3) Pathologically confirmed HER2-low expressing, unresectable locally advanced or metastatic breast cancer;
   a) Low HER2 expression (IHC 2+ and ISH-, or IHC 1+) confirmed by pathological test results from a central laboratory;
   b) If multiple prior HER2 pathological results exist, the specimen submitted to the central laboratory must be derived from the most recent tumor tissue pathological specimen in the recurrent and/or metastatic stage of breast cancer, and the specimen must meet the pathological diagnosis of low HER2 expression;
   c) Prior pathological reports have never diagnosed HER2-positive (IHC 3+ or ISH+) breast cancer;
(4) Hormone receptor (HR) status has been clearly identified; if the subject has multiple ER/PR results in the recurrent and/or metastatic stage, the most recent test results shall be used to confirm eligibility;
(5) Prior treatment for subjects must meet the following requirements:
   a) No prior systemic chemotherapy for breast cancer in the recurrent and/or metastatic stage has been received; development of progressive disease during (neo)adjuvant chemotherapy or within 12 months after the end of (neo)adjuvant chemotherapy is considered as having received first-line chemotherapy;
   b) Subjects with HR-positive breast cancer have received at least one line of endocrine therapy in the recurrent and/or metastatic stage and developed progressive disease, or have developed progressive disease within 24 months from the start of adjuvant endocrine therapy, and meanwhile, the investigator determines that the subject cannot continue to benefit from endocrine therapy (note: if progressive disease develops more than 24 months after the start of adjuvant endocrine therapy, it is not considered as first-line endocrine therapy in the recurrent and/or metastatic stage);
   c) Subjects with HR-positive breast cancer receiving CDK4/6 inhibitors, HDAC inhibitors, PARP inhibitors, or PI3K/mTOR/AKT inhibitors are classified as receiving targeted therapy, and monotherapy is not counted as a line of endocrine therapy;
   d) HR-negative breast cancer subjects with known positive PD-L1 expression (CPS ≥ 1) cannot be enrolled;
   e) The number of lines of prior systemic anti-tumor treatment is all defined by progressive disease; when no progressive disease development has occurred, changing drug treatment or adding new anti-tumor drugs on the basis of the original drugs is still considered as the same line of therapy;
(6) Radiologically confirmed progressive disease (during or after the most recent treatment);
(7) Having at least one measurable lesion according to RECIST 1.1 criteria (except for cases with only skin and/or brain lesions as measurable lesions);
(8) Good function of major organs, meeting the criteria for blood routine examination, biochemical examination, coagulation function examination, and cardiac color ultrasound assessment;
(9) Female subjects of childbearing age should agree to take necessary contraceptive measures (such as intrauterine devices, contraceptives, or condoms) during the study and for 6 months after the study; the serum pregnancy test results should be negative within 7 days before enrollment, and the subjects must be in the non-lactation period; male subjects should agree to take necessary contraceptive measures during the study and for 6 months after the study.

### 3. Test drug

Test group: ADC1 for injection (specification: 100 mg/vial), developed and provided by Chia Tai Tianqing Pharmaceutical Group Shunxin Pharmaceutical Co., Ltd.

Control group: chemotherapy regimens selected by the investigator; optional chemotherapeutic drugs include capecitabine, paclitaxel, and albumin paclitaxel, all of which are developed and provided by Chia Tai Tianqing Pharmaceutical Group Shunxin Pharmaceutical Co., Ltd.

### 4. Treatment regimen

Test group: ADC1 was administered via intravenous infusion once every 3 weeks at a dose of 7.5 mg/kg each time, with 21 days as one treatment cycle.

Control group: (1) capecitabine: oral administration, twice daily, at a dose of 1000-1250 mg/m² each time, continuously administered from day 1 to day 14, with 21 days as one treatment cycle; (2) paclitaxel: intravenous infusion, once every 3 weeks, at a dose of 175 mg/m² each time, with 21 days as one treatment cycle; (3) albumin paclitaxel: intravenous infusion, once every 3 weeks, at a dose of 260 mg/m² each time, with 21 days as one treatment cycle; or intravenous infusion, administered once on day 1 and once on day 8, at a dose of 100 mg/m² or 125 mg/m² each time, with 21 days as one treatment cycle; or intravenous infusion, administered once on day 1, once on day 8, and once on day 15, at a dose of 100 mg/m² or 125 mg/m² each time, with 21 days as one treatment cycle.

### 5. Evaluation criteria

Efficacy evaluation: the disease status was determined using the RECIST 1.1 criteria.

Safety evaluation: the severity of adverse events was determined using the NCI-CTC AE 5.0 criteria.

### 6. Endpoint indicators

### 6.1 Primary endpoint

Progression-free survival (PFS) of subjects with HR-positive and HER2-low expressing recurrent and/or metastatic breast cancer as assessed by the Independent Review Committee (IRC).

### 6.2 Key secondary endpoint

Progression-free survival (PFS) of subjects with HER2-low expressing recurrent and/or metastatic breast cancer as assessed by the IRC.

### 6.3 Secondary endpoints

Progression-free survival (PFS), overall survival (OS), duration of response (DOR), objective response rate (ORR), and clinical benefit rate (CBR) of the HR-positive population with low HER2 expression as assessed by the investigator;
Progression-free survival (PFS), overall survival (OS), duration of response (DOR), objective response rate (ORR), and clinical benefit rate (CBR) of the population with low HER2 expression as assessed by the investigator;
Incidence and severity of adverse events (AEs) and serious adverse events (SAEs), and abnormal laboratory test indicators;
Plasma concentrations of the ADC drug, total antibody, and cytotoxic drug of ADC1;
Immunogenicity of ADC1: such as ADA incidence.

### 6.4 Exploratory endpoint

Progression-free survival (PFS), overall survival (OS), duration of response (DOR), objective response rate (ORR), and clinical benefit rate (CBR) of the HR-negative population with low HER2 expression.

In this study, the use of ADC1 can achieve desirable clinical benefits.

## Claims

1. An anti-HER2 antibody-drug conjugate for use in treating breast cancer in a subject, wherein the anti-HER2 antibody-drug conjugate is formed by linking a drug-linker having a structure represented by formula Ia below 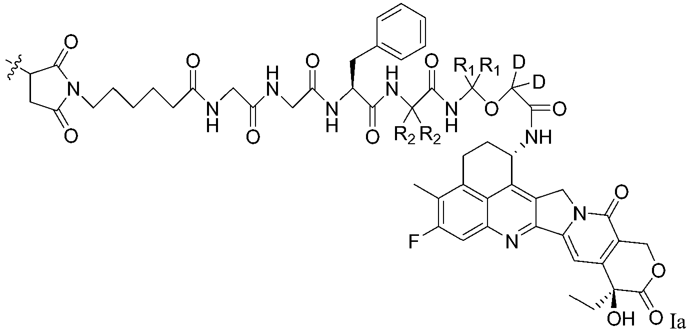
to an HER2-targeting antigen-binding construct, wherein
R₁ and R₂ in formula Ia are each independently selected from hydrogen and deuterium; preferably, R₁ and R₂ are hydrogen;
position 3 of -(succinimidyl-3-yl-N)- is linked to the HER2-targeting antigen-binding construct;
the HER2-targeting antigen-binding construct comprises a first antigen-binding fragment and a second antigen-binding fragment;
the first antigen-binding fragment comprises: an HCDR1 comprising the amino acid sequence set forth in SEQ ID NO: 1, an HCDR2 comprising the amino acid sequence set forth in SEQ ID NO: 2, an HCDR3 comprising the amino acid sequence set forth in SEQ ID NO: 3, an LCDR1 comprising the amino acid sequence set forth in SEQ ID NO: 4, an LCDR2 comprising the amino acid sequence set forth in SEQ ID NO: 5, and an LCDR3 comprising the amino acid sequence set forth in SEQ ID NO: 6;
the second antigen-binding fragment comprises: an HCDR1 comprising the amino acid sequence set forth in SEQ ID NO: 9, an HCDR2 comprising the amino acid sequence set forth in SEQ ID NO: 10, an HCDR3 comprising the amino acid sequence set forth in SEQ ID NO: 11, an LCDR1 comprising the amino acid sequence set forth in SEQ ID NO: 12, an LCDR2 comprising the amino acid sequence set forth in SEQ ID NO: 13, and an LCDR3 comprising the amino acid sequence set forth in SEQ ID NO: 14.

2. The anti-HER2 antibody-drug conjugate for use in treating breast cancer in a subject according to claim 1, wherein an average number of linked drug-linkers per HER2-targeting antigen-binding construct is 4 to 7, 5 to 6, or 5.5 to 6.

3. The anti-HER2 antibody-drug conjugate for use in treating breast cancer in a subject according to claim 1 or 2, wherein
(i) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7;
(ii) the first antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
(iii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8;
(iv) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15;
(v) the second antigen-binding fragment comprises a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16;
(vi) the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16; or
(vii) the first antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 7, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 8; and, the second antigen-binding fragment comprises a heavy chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 15, and a light chain variable region having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 16.

4. The anti-HER2 antibody-drug conjugate for use in treating breast cancer in a subject according to any one of claims 1-3, wherein
(i) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20;
(ii) the HER2-targeting antigen-binding construct comprises a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21;
(iii) the HER2-targeting antigen-binding construct comprises a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19; or
(iv) the HER2-targeting antigen-binding construct comprises a first polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 17 or 20, a second polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 18 or 21, and a third polypeptide chain having an amino acid sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to the amino acid sequence set forth in SEQ ID NO: 19.

5. The anti-HER2 antibody-drug conjugate for use in treating breast cancer in a subject according to any one of claims 1-4, wherein the anti-HER2 antibody-drug conjugate is administered to the subject at a dose of 1.5-9 mg/kg, 4.5-7.5 mg/kg, or 6-7.5 mg/kg each time; or, the anti-HER2 antibody-drug conjugate is administered to the subject at a dose of 1.5 mg/kg, 3 mg/kg, 4.5 mg/kg, 6 mg/kg, 7.5 mg/kg, or 9 mg/kg each time.

6. The anti-HER2 antibody-drug conjugate for use in treating breast cancer in a subject according to any one of claims 1-5, wherein the anti-HER2 antibody-drug conjugate is administered once every 1 week, every 2 weeks, every 3 weeks, or every 4 weeks.

7. The anti-HER2 antibody-drug conjugate for use in treating breast cancer in a subject according to any one of claims 1-6, wherein the anti-HER2 antibody-drug conjugate is formulated into a formulation for intravenous injection or infusion.

8. The anti-HER2 antibody-drug conjugate for use in treating breast cancer in a subject according to any one of claims 1-7, wherein the breast cancer is recurrent and/or metastatic breast cancer or locally advanced or metastatic breast cancer.

9. The anti-HER2 antibody-drug conjugate for use in treating breast cancer in a subject according to any one of claims 1-8, wherein the breast cancer is unresectable breast cancer;
the breast cancer is HER2-expressing breast cancer; and/or
the breast cancer is hormone receptor-positive breast cancer.

10. The anti-HER2 antibody-drug conjugate for use in treating breast cancer in a subject according to any one of claims 1-9, wherein the breast cancer is HER2-positive breast cancer or HER2-low expressing breast cancer; the breast cancer is estrogen receptor- or/and progesterone receptor-positive breast cancer; or
the breast cancer is hormone receptor-negative or positive and HER2-positive recurrent or metastatic breast cancer;
wherein the HER2-positive breast cancer is breast cancer determined to have HER2 expression of 3+ by IHC, or the HER2-positive breast cancer is breast cancer determined to have HER2 expression of 2+ by IHC and determined to be positive for HER2 expression by ISH;
and wherein the HER2-low expressing breast cancer is breast cancer determined to have HER2 expression of 2+ by IHC and determined to be negative for HER2 expression by ISH, or the HER2-low expressing breast cancer is breast cancer determined to have HER2 expression of 1+ by IHC.

11. The anti-HER2 antibody-drug conjugate for use in treating breast cancer in a subject according to any one of claims 1-10, wherein the subject with breast cancer is unsuitable for receiving surgery and/or radiation therapy; the subject with breast cancer has previously received at least one line of treatment for treating breast cancer; the subject with breast cancer has previously received adjuvant therapy or neoadjuvant therapy for treating breast cancer;
the subject with breast cancer has previously received local radiation therapy for treating breast cancer;
the subject with breast cancer has previously received anti-HER2 antibody treatment, a taxane drug, a CDK4/6 inhibitor, endocrine therapy, and/or adjuvant endocrine therapy; and/or
the subject with breast cancer has not received chemotherapy in the recurrent and/or metastatic stage.

12. The anti-HER2 antibody-drug conjugate for use in treating breast cancer in a subject according to any one of claims 1-11, wherein the subject with breast cancer is a subject with HER2-expressing recurrent and/or metastatic breast cancer who has received and then failed at least one line of systemic chemotherapy in the recurrent and/or metastatic stage;
the subject with breast cancer is a hormone receptor-positive subject with HER2-expressing recurrent and/or metastatic breast cancer who has received and then failed the treatment of a CDK4/6 inhibitor in combination with endocrine therapy in the recurrent and/or metastatic stage;
the subject with breast cancer is a subject with HER2-expressing recurrent and/or metastatic breast cancer who has received the treatment with trastuzumab and a taxane drug;
the subject with breast cancer is a subject with HER2-low expressing locally advanced or metastatic breast cancer who has not received chemotherapy in the recurrent and/or metastatic stage; or
the subject with breast cancer is a subject with HR-positive and HER2-low expressing locally advanced or
metastatic breast cancer who has received at least one line of endocrine therapy in the recurrent and/or metastatic stage and developed progressive disease, or who has developed progressive disease within 24 months after the start of adjuvant endocrine therapy.

13. The anti-HER2 antibody-drug conjugate for use in treating breast cancer in a subject according to any one of claims 1-12, wherein the subject with breast cancer is a subject with HER2-positive recurrent and/or metastatic breast cancer who has received and then failed at least one line of systemic chemotherapy in the recurrent and/or metastatic stage;
the subject with breast cancer is a subject with HER2-positive recurrent and/or metastatic breast cancer who has received the treatment with trastuzumab and a taxane drug;
the subject with breast cancer is a subject with HER2-low expressing recurrent and/or metastatic breast cancer who has received and then failed at least one line of systemic chemotherapy in the recurrent and/or metastatic stage;
the subject with breast cancer is a hormone receptor-positive subject with HER2-low expressing recurrent and/or metastatic breast cancer who has received and then failed the treatment of a CDK4/6 inhibitor in combination with endocrine therapy in the recurrent and/or metastatic stage;
the subject with breast cancer is a subject with HER2-low expressing unresectable locally advanced or metastatic breast cancer who has not received chemotherapy in the recurrent and/or metastatic stage; or
the subject with breast cancer is a subject with HR-positive and HER2-low expressing unresectable locally advanced or metastatic breast cancer who has received at least one line of endocrine therapy in the recurrent and/or metastatic stage and developed progressive disease, or who has developed progressive disease within 24 months after the start of adjuvant endocrine therapy.

14. The anti-HER2 antibody-drug conjugate for use in treating breast cancer in a subject according to any one of claims 1-13, wherein the anti-HER2 antibody-drug conjugate is for use in treating breast cancer in a subject in second-line treatment or a later line of treatment.

15. The anti-HER2 antibody-drug conjugate for use in treating breast cancer in a subject according to any one of claims 1-14, wherein the anti-HER2 antibody-drug conjugate is formulated, together with one or more pharmaceutically acceptable excipients, into a pharmaceutical composition, and the pharmaceutical composition comprises a therapeutically effective amount of the anti-HER2 antibody-drug conjugate.
